(19) Europäisches Patentamt / European Patent Office / Office européen des brevets

(11) **EP 1 958 567 B1**

(12) **EUROPEAN PATENT SPECIFICATION**

(45) Date of publication and mention
of the grant of the patent:
**02.01.2013 Bulletin 2013/01**

(21) Application number: **06810059.3**

(22) Date of filing: **12.09.2006**

(51) Int Cl.:
***A61B 5/053*** (2006.01)

(86) International application number:
**PCT/JP2006/318042**

(87) International publication number:
**WO 2007/063631 (07.06.2007 Gazette 2007/23)**

(54) **BODY FAT MEASURING APPARATUS CAPABLE OF EASILY AND ACCURATELY MEASURING AMOUNT OF VISCERAL FAT**

KÖRPERFETT-MESSGERÄT ZUR LEICHTEN UND GENAUEN MESSUNG DER VISZERALEN FETTMENGE

APPAREIL DE MESURE DE LA GRAISSE CORPORELLE CAPABLE DE MESURER FACILEMENT ET PRÉCISÉMENT LA QUANTITÉ DE GRAISSE VISCÉRALE

(84) Designated Contracting States:
**DE ES FR GB IT**

(30) Priority: **30.11.2005 JP 2005345917**

(43) Date of publication of application:
**20.08.2008 Bulletin 2008/34**

(73) Proprietor: **Omron Healthcare Co., Ltd.
Kyoto 615-0084 (JP)**

(72) Inventors:
• **OKU, Shojiro
c/o Omron Healthcare Co., Ltd.
Kyoto 615-0084 (JP)**
• **SHIGA, Toshikazu
c/o Omron Healthcare Co., Ltd.
Kyoto 615-0084 (JP)**
• **KUBO, Nobuo
c/o Omron Healthcare Co., Ltd.
Kyoto 615-0084 (JP)**
• **TOGOE, Yasuyuki
c/o Omron Healthcare Co., Ltd.
Kyoto 615-0084 (JP)**

(74) Representative: **Wilhelms · Kilian & Partner
Patentanwälte
Eduard-Schmid-Straße 2
81541 München (DE)**

(56) References cited:
EP-A1- 1 707 117          EP-A1- 1 731 092
EP-A1- 1 741 385          WO-A1-2004/030535
WO-A2-2005/010640          JP-A- 11 123 182
JP-A- 2000 051 369          JP-A- 2001 252 257
JP-A- 2002 238 870          JP-A- 2002 369 806
JP-A- 2003 230 547          JP-A- 2005 103 198
US-A1- 2004 059 242          US-B1- 6 473 642

**Description**

TECHNICAL FIELD

The present invention relates to a body fat measuring apparatus and an upper limb unit, and particularly to a body fat measuring apparatus and an upper limb unit capable of measuring a visceral fat amount using an impedance method.

BACKGROUND ART

Conventionally, the visceral fat amount is actually measured using a tomogram of the abdomen taken with X-ray CT (Computed Tomography). Therefore, a problem here is that the visceral fat amount can be measured at only those medical institutions having X-ray CT facilities. While the MRI (Magnetic Resonance Imaging) can also be used to take a tomogram of the abdomen like that with the X-ray CT, large-sized facilities are necessary as well. Therefore, subjects cannot easily and conveniently measure the visceral fat amount by themselves at their home.
Accordingly, as a method of easily and conveniently measuring the visceral fat amount, recently an impedance method has been proposed.
For example, Japanese Patent Laying-Open No. 07-079938 (hereinafter Patent Document 1) discloses that the visceral fat amount is calculated based on specific physical data of a subject and the impedances of the four limbs. Japanese Patent Laying-Open No. 2000-152919 (hereinafter Patent Document 2) discloses that conductive contact with the hands and abdomen are made to apply an electric current between the hands and abdomen, take the human body impedance and thereby measure the body fat percentage of the upper half of the body. In this way, it can be known whether the obesity is subcutaneous fat obesity or visceral fat obesity. Japanese Patent Laying-Open No. 2002-369806 (hereinafter Patent Document 3) discloses the technique of calculating the visceral fat amount based on physical data (such as waist length and gender) of a person whose measurement is taken and the voltage value of the abdomen.
A proposal has also been made for allowing an electrode for the abdomen, which is provided for measuring the impedance of the abdomen, to be easily set against the abdomen of the subject by him or herself.
Patent Document 3 as described above discloses that the subject winds a belt provided with a plurality of electrodes arranged in advance around the abdomen to allow the electrodes to contact the abdomen.
Further, as a positioning technique, Japanese Patent Laying-Open No. 2002-282241 (hereinafter Patent Document 4) discloses that a base body which is an apparatus's main unit held with hands is provided with a navel position setting portion for positioning the apparatus using the navel of the subject as a reference position. As the navel position setting portion, a rod-shaped body is shown that is fit in a hole passing through the base body from the front surface to the rear surface.
Patent Document 1: Japanese Patent Laying-Open No. 07-079938
Patent Document 2: Japanese Patent Laying-Open No. 2000-152919
Patent Document 3: Japanese Patent Laying-Open No. 2002-369806
Patent Document 4: Japanese Patent Laying-Open No. 2002-282241
EP 1 707 117 EP 1 731 092, EP 1 741 385 are prior art under Article 54(3) EPC and disclose body fat measuring devices comprising electrodes for measuring body impedance from the hands, the abdomen and the feet and means for calculating a visceral fat quantity on the basis of measured impedances.

DISCLOSURE OF THE INVENTION

PROBLEMS TO BE SOLVED BY THE INVENTION

Patent Documents 1 to 3, however, do not disclose that the visceral fat amount is calculated using information about the subcutaneous fat amount as an important or necessary factor. Therefore, the influence of the subcutaneous fat cannot be avoided, resulting in a problem that the visceral fat amount cannot be measured accurately. Further, Patent Document 4 does not disclose that the visceral fat amount is determined.
In addition, regarding the apparatus disclosed in Patent Document 4, the subject has to place the rod-shaped navel position setting portion at the navel of the subject using the subject's senses.
The present invention has been made to solve the problems as described above, and an object of the invention is to provide a body fat measuring apparatus and an upper limb unit capable of easily and conveniently measuring the visceral fat amount with accuracy.

MEANS FOR SOLVING THE PROBLEMS

According to an aspect of the present invention, a body fat measuring apparatus is defined in claim 1 and includes an upper limb unit including a hold portion that can be held in a hand of a subject, and a main portion. The hold portion includes an upper limb electrode to be contacted with a palm part of the subject, and the main portion includes a first surface to be contacted with a surface of an abdomen of the subject, and a group of abdomen electrodes arranged at the first surface. The group of abdomen electrodes includes a pair of first electrodes and a pair of second electrodes. The body fat measuring apparatus further includes: a lower limb electrode to be contacted with a lower limb of the subject; an applying portion for applying an electric current to the subject via one of a pair of the upper limb electrode and the lower limb electrode and the pair of the second electrodes; and a detecting portion for detecting two types of potential differences respectively in a first case where the electric current is applied via the pair of the upper limb electrode and the lower limb electrode and in a second case where the electric current is applied via the pair of the second electrodes. The detecting portion detects a first potential difference between electrodes of a predetermined pair included in the group of electrodes in the first case, and a second potential difference between the first electrodes in the second case. The body fat measuring apparatus further includes a visceral fat amount calculating portion for calculating a visceral fat amount of the subject based on the first potential difference and the second potential difference that are detected and physical data of the subject.

Here, "abdomen" refers to a portion of the body trunk except for the thorax. Further, "visceral fat amount" refers to an amount concerning visceral fat and includes for example at least one of visceral fat weight, visceral fat area and visceral fat volume.

Preferably, the first electrodes and the second electrodes are arranged at the first surface in an alignment direction that is a vertical direction in a state where the subject takes a posture for taking a measurement and holds the hold portion.

Preferably, the second electrodes are arranged at respective positions sandwiching the first electrodes in the alignment direction.

Preferably, the electrodes of the predetermined pair are the first electrodes.

Alternatively, the electrodes of the predetermined pair may be a pair of electrodes other than the pair of the first electrodes included in the group of abdomen electrodes.

Preferably, the second electrodes are arranged at respective positions sandwiching the first electrodes in the alignment direction, and the electrodes of the predetermined pair are the second electrodes.

Preferably, the visceral fat measuring apparatus further includes an impedance calculating portion for calculating two types of impedances based on the first potential difference and the second potential difference respectively, and the visceral fat amount calculating portion calculates the visceral fat amount based on the two types of impedances that are calculated and the physical data of the subject.

Preferably, the visceral fat amount calculating portion calculates the visceral fat amount using a predetermined correlation formula of a relation between the two types of impedances, the physical data of the subject and the visceral fat amount.

Preferably, the visceral fat measuring apparatus further includes a display portion for displaying the visceral fat amount as calculated.

Preferably, the visceral fat measuring apparatus further includes a subcutaneous fat amount calculating portion for calculating a subcutaneous fat amount of the subject based on the second potential difference as detected and the physical data of the subject.

"Subcutaneous fat amount" refers to an amount concerning subcutaneous fat, and includes for example at least one of subcutaneous fat weight, subcutaneous fat area and subcutaneous fat volume.

Preferably, the physical data includes at least one of circumference of the abdomen, lateral width of the abdomen, thickness of the abdomen, height, and weight of the subject.

Here, "circumference of the abdomen" refers to the length of the circumference of a cross section of the abdomen and preferably refers to the length of the circumference of a cross section of a middle portion (around the navel) of the abdomen. It is also called waist length. "Lateral width of the abdomen" refers to the right-to-left width (length) of the abdomen and preferably refers to the right-to-left width of a middle portion of the abdomen. "Thickness of the abdomen" refers to the front-to-back thickness (length) of the abdomen and preferably refers to the front-to-back thickness of a middle portion of the abdomen.

Preferably, the visceral fat measuring apparatus further includes a lower limb unit where a foot of the subject can be placed, and the lower limb electrode is provided at the lower limb unit.

Preferably, the main portion further includes positioning means corresponding to a navel position of the subject.

Preferably, the positioning means is formed by a depression provided in an upper end portion of the first surface. Alternatively, the positioning means preferably includes a camera provided at the first surface, and a display portion provided at the main portion for displaying an object whose image is shot by the camera. Alternatively, the positioning means is preferably formed by a mirror provided at the first surface. Alternatively, the positioning means is preferably formed by an irradiating apparatus provided at the first surface for applying laser light.

Further, the main portion further includes a second surface opposite to the first surface, and the positioning means may be a hole passing through from the first surface toward the second surface. Alternatively the positioning means may be a window providing visibility from the second surface toward the first surface.

According to another aspect of the present invention, an upper limb unit for measuring body fat of a subject includes: a hold portion that can be held in a hand of the subject, the hold portion including an upper limb electrode to be contacted with a palm part of the subject; and a main portion. The main portion includes: a surface to be contacted with a surface of an abdomen of the subject; and a group of abdomen electrodes arranged at the surface, and the group of abdomen electrodes includes a pair of first electrodes and a pair of second electrodes. The main portion further includes: an applying portion for applying an electric current to the subject via one of a pair of a lower limb electrode contacted with a lower limb of the subject and the upper limb electrode and the pair of the second electrodes; and a detecting portion for detecting two types of potential differences respectively in a first case where the electric current is applied via the pair of the upper limb electrode and the lower limb electrode and in a second case where the electric current is applied via the pair of the second electrodes. The detecting portion detects a first potential difference between electrodes of a predetermined pair included in the group of electrodes in the first case, and a second potential difference between the first electrodes in the second case. The main portion further includes a visceral fat amount calculating portion for calculating a visceral fat amount of the subject based on the first potential difference and the second potential difference that are detected and physical data of the subject.

EFFECTS OF THE INVENTION

In accordance with the present invention, the visceral fat amount can be easily and conveniently measured with accuracy.

BRIEF DESCRIPTION OF THE DRAWINGS

Fig. 1 shows an example of an external appearance of a body fat measuring apparatus according to a first embodiment of the present invention.
Fig. 2A illustrates how the body fat is measured using the body fat measuring apparatus in the first embodiment, as seen from the front side of a subject.
Fig. 2B illustrates how the body fat is measured using the body fat measuring apparatus, as seen from a lateral side of a subject.
Fig. 3 is a block diagram showing a hardware configuration of the body fat measuring apparatus in the first embodiment of the present invention.
Fig. 4 is a block diagram showing a functional configuration of an operation processing portion.
Fig. 5 is a schematic perspective view of an upper limb unit of the body fat measuring apparatus in the first embodiment of the present invention.
Fig. 6 is a flowchart showing a body fat measuring process in the first embodiment of the present invention.
Fig. 7 shows an example where measurement results are displayed.
Fig. 8A shows an example where only a visceral fat area is displayed.
Fig. 8B shows an example where a visceral fat area and a subcutaneous fat area are displayed.
Fig. 9A is a first diagram showing an example where data concerning a subject is further displayed in addition to measurement results.
Fig. 9B is a second diagram showing an example where data concerning a subject is further displayed in addition to measurement results.
Fig. 10A is a diagram showing an example where a reference value of the visceral fat area is further displayed together with measurement results.
Fig. 10B is a diagram showing an example where a standard value of the same gender as and of similar age to a subject is further displayed together with measurement results.
Fig. 11 is a diagram showing an example of the case where measured values taken in the past are displayed in the form of a graph together with measurement results.
Fig. 12A shows another example of a positioning index in the first embodiment.
Fig. 12B shows still another example of the positioning index in the first embodiment.
Fig. 13 is a schematic front view of an upper limb unit of a body fat measuring apparatus in a second embodiment of the present invention.

EP 1 958 567 B1

Fig. 14 is a block diagram showing a hardware configuration of the body fat measuring apparatus in the second embodiment of the present invention.

Fig. 15 is a flowchart showing a body fat measuring process in the second embodiment of the present invention.

Fig. 16 is a flowchart showing the body fat measuring process in the second embodiment of the present invention.

Fig. 17A shows an example of a positioning index in the second embodiment of the present invention.

Fig. 17B shows another example of the positioning index in the second embodiment of the present invention.

Fig. 18 is a schematic front view of an upper limb unit 1B of a body fat measuring apparatus in a third embodiment of the present invention.

Fig. 19 is a block diagram showing a hardware configuration of the body fat measuring apparatus in the third embodiment of the present invention.

Fig. 20 is a flowchart showing a body fat measuring process in the third embodiment of the present invention.

Fig. 21 shows an example of a positioning index in the third embodiment of the present invention.

DESCRIPTION OF THE REFERENCE SIGNS

1, 1A, 1B upper limb unit, 2 lower limb unit, 3 cable, 12 constant current generating portion, 13 voltage detecting portion, 14, 14A, 14B terminal switching portion, 16a, 16b connector, 20 control portion, 21 display portion, 22 input portion, 23b recording medium, 23a drive device, 26, 26A, 26B operation processing portion, 27 storage portion, 31 measurement start key, 32 measurement stop key, 33 hinge, 100, 100A, 100B body fat measuring apparatus, 110, 110A, 110B main portion, 111 first housing, 112, 112A, 112B second housing, 121, 122 grip, 130, 130A, 130B surface, 203 camera, 204 light, 206 laser LED, 211 groove, 212 mirror, 221 window, 261 impedance calculating portion, 262 body fat calculating portion, 262C body fat amount calculating portion, 262A visceral fat amount calculating portion, 262B subcutaneous fat amount calculating portion, A, B, C, D, A11, B11, C11, D11, A12, B12, C12, D12, A21, B21, C21, D21, A22, B22, C22, D22, F1, F2, F3, F4, H1, H2, H3, H4 electrode

BEST MODES FOR CARRYING OUT THE INVENTION

Embodiments of the present invention will be described in detail with reference to the drawings. In the drawings, like or corresponding components are denoted by like reference characters.

[First Embodiment]

<External Appearance and Configuration of Body Fat Measuring Apparatus>

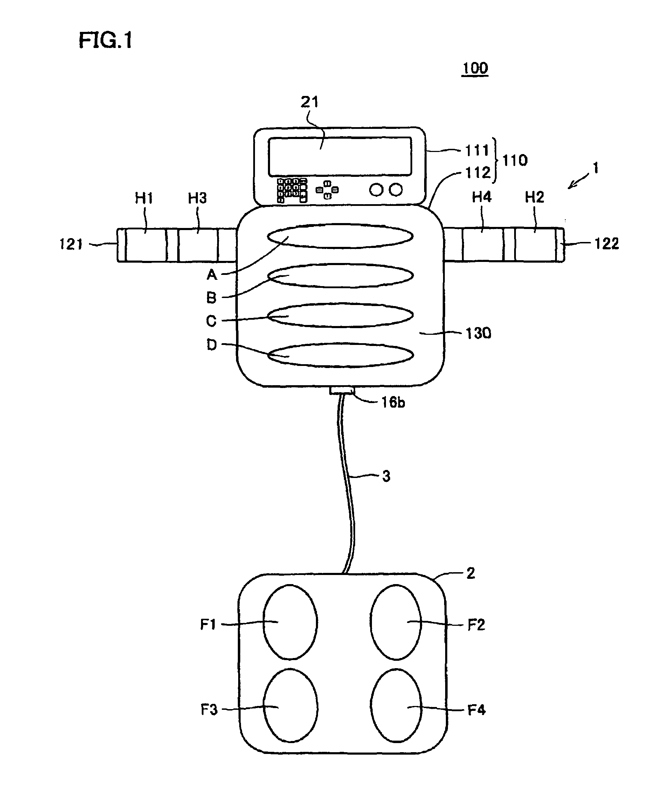

As to external appearance:
Referring to Fig. 1, a body fat measuring apparatus 100 in a first embodiment of the present invention includes an upper limb unit 1, a lower limb unit 2 and a cable 3 for electrically connecting upper limb unit 1 and lower limb unit 2.
Upper limb unit 1 includes a main portion 110 and grips 121, 122 that can be held in both left and right hands of a subject. Grip 121 is a grip for the left hand and includes electrodes H1, H3 to be contacted with the left palm of the subject. Grip 122 is a grip for the right hand and includes electrodes H2, H4 to be contacted with the right palm of the subject. Electrodes H1, H2 are electrodes for applying an electric current, and electrodes H3, H4 are electrodes for detecting a voltage.
Main portion 110 includes a surface 130 to be contacted with the surface of the abdomen of the subject and electrodes A, B, C, D arranged at surface 130. In the present embodiment, surface 130 is planar-shaped. However, the surface may be shaped in a curve along the surface of the abdomen.
Preferably, electrodes A, B, C, D are arranged in an alignment direction that is the vertical direction in the state where the subject is in a posture for taking a measurement and holds grips 121, 122. The shape and size of the electrodes and the interval between the electrodes are not limited to particular ones. It is sufficient that all of the electrodes are arranged such that the electrodes can contact the surface of the abdomen of the subject.
In the present embodiment, main portion 110 is formed of a first housing 111 and a second housing 112. Surface 130 as described above is provided to second housing 112. Further, first housing 111 is provided with at least a display portion 21.
Lower limb unit 2 includes electrodes F1, F2, F3, F4 to be contacted with lower limbs of the subject. Electrodes F1, F2 are used for applying an electric curent and electrodes F3, F4 are used for detecting a voltage.
In the present embodiment, upper limb unit 1 is structured such that the upper limb unit is attachable to and detachable from cable 3 connected to lower limb unit 2. For example, an end of cable 3 is provided with a connector 16b used for allowing attachment to a connector 16a provided in upper limb unit 1.
In the following description, electrodes H1, ... , H4 are collectively referred to as "upper limb electrode," electrodes A, ... , D are collectively referred to as "abdomen electrode" and electrodes F1, ... , F4 are collectively referred to as "lower limb electrode." Further, the upper limb electrode, the abdomen electrode and the lower limb electrode are collectively referred to as "electrode group."

Here, a posture taken by the subject using body fat measuring apparatus 100 will be described.

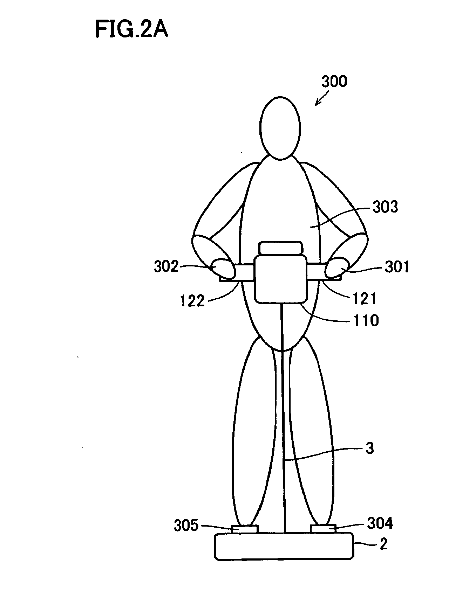

Referring to Fig. 2A which illustrates how the body fat is measured using body fat measuring apparatus 100 in the first embodiment of the present invention, as seen from the front side of a subject, grip 121 is held in a left hand 301 of a subject 300, and grip 122 is held in a right hand 302 of subject 300. Accordingly, electrodes H1, H3 and electrodes H2, H4 are respectively contacted with left hand 301 and right hand 302 of subject 300. Left and right feet 304, 305 of subject 300 are set on the upper surface of lower limb unit 2. Accordingly, electrodes F1, F3 and electrodes F2, F4 are respectively contacted with left foot 304 and right foot 305 of subject 300.

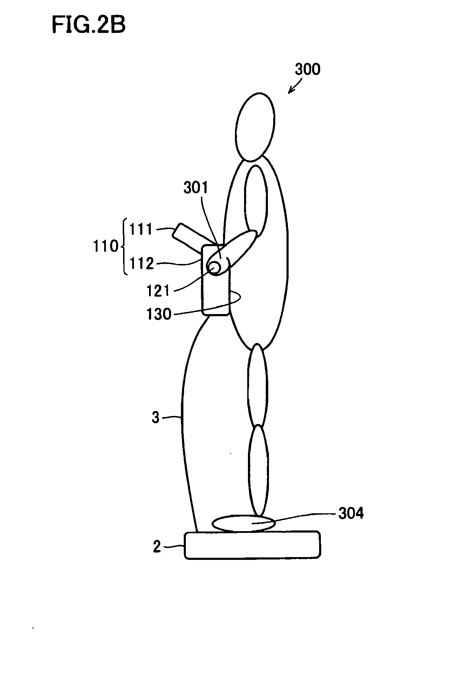

Referring to Fig. 2B which illustrates how the body fat is measured using body fat measuring apparatus 100 in the first embodiment of the present invention, as seen from a lateral side of the subject, surface 130 of main portion 110 is pressed against the surface of an abdomen 303 of subject 300. Accordingly, the abdomen electrodes are caused to contact the surface of abdomen 303. As described above, in the present embodiment, the abdomen electrodes are arranged in an alignment direction that is the vertical direction in the state where subject 300 is in a posture for taking a measurement and holds grips 121, 122. Therefore, the abdomen electrodes contact the surface of abdomen 303 in the alignment direction that is the direction substantially perpendicular to a cross section of abdomen 303 of subject 300 (the direction is hereinafter referred to as "lengthwise direction").

As to configuration:

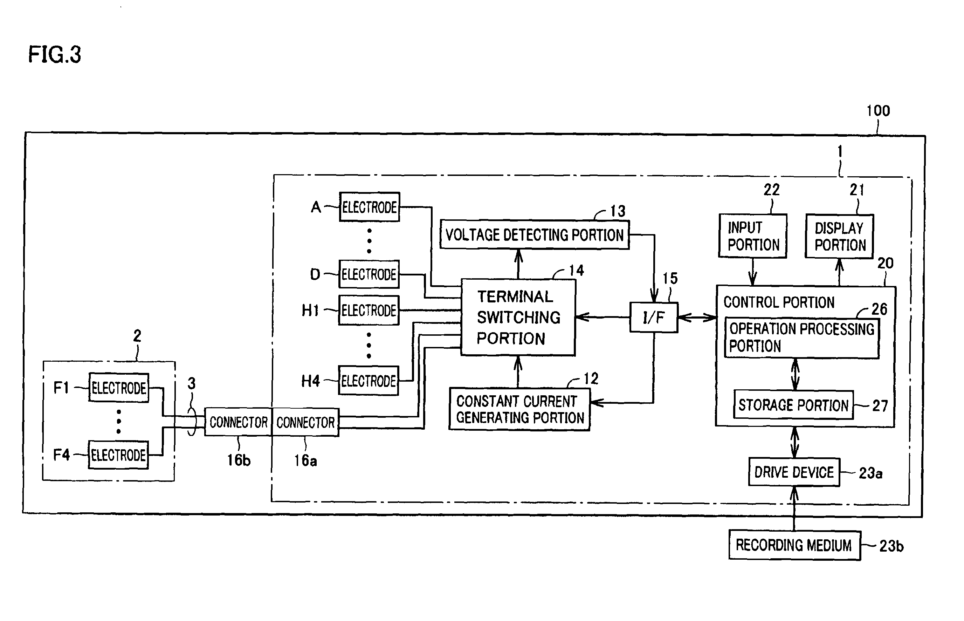

Referring to Fig. 3, upper limb unit 1 of body fat measuring apparatus 100 in the first embodiment of the present invention includes, in addition to the above-described upper limb electrode, the abdomen electrode and connector 16a, a constant current generating portion 12 for generating a high-frequency constant current (50 kHz, 500 μA for example), a voltage detecting portion 13 for detecting a potential difference between electrodes of one pair to which the electric current is applied, a terminal switching portion 14 for selecting an electrode for the current and an electrode for the voltage from the electrode group, a control portion 20 performing overall control of body fat measuring apparatus 100, a display portion 21 for displaying results of the measurement for example, an input portion 22 for entering subject data as described hereinlater, a drive device 23 a capable of reading and writing data recorded by a recording medium 23b, and an I/F (interface) 15 for making communication between control portion 20 and constant current generating portion 12, voltage detecting portion 13, terminal switching portion 14.

Here, "subject data" refers to data concerning the body of the subject including at least physical data, and includes for example, at least one of such data as the waist length (the circumference of the abdomen), lateral width of the abdomen, thickness of the abdomen, height, weight, age, and gender. In the present embodiment, the physical data is described as the data corresponding to the waist length.

The physical data is not limited to the waist length and may be other data concerning the abdomen (such as lateral width of the abdomen, thickness of the abdomen) or data concerning the whole body (such as height, weight). The physical data is not limited to a piece of data and may include two or more pieces of data.

Terminal switching portion 14 is connected to constant current generating portion 12 and voltage detecting portion 13 and connected to each electrode included in the electrode group. Terminal switching portion 14 is controlled by control portion 20 to switch electrodes and select at least one pair of electrodes for the current from the electrode group. Thus, the constant current generated by constant current generating portion 12 is applied via the selected electrodes to the subject. Terminal switching portion 14 is also controlled by control portion 20 to switch electrodes and select a pair of electrodes for the voltage from the electrode group. Thus, voltage detecting portion 13 can detect a potential difference between the electrodes selected each time. The information about the detected potential difference is supplied via I/F 15 to control portion 20. Terminal switching portion 14 is formed for example of a plurality of switches.

Here, constant current generating portion 12 and terminal switching portion 14 are used to apply the current to the subject via one of the two pairs of electrodes H1, H2 and F1, F2 and one pair of electrodes A, D. Further, in any of the cases, in the state where the current is applied to the subject, a potential difference between a pair of electrodes B and C is detected by voltage detecting portion 13 and terminal switching portion 14.

Control portion 20 includes an operation processing portion 26 for performing various types of operation and a storage portion 27 for storing a program and data. Here, a body fat measuring program recorded by recording medium 23b may be read by drive device 23 a and a body fat measuring process as described below may be performed.

Display portion 21 is formed for example of a liquid crystal. Input portion 22 is formed for example of a plurality of keys that can be pressed by a user. A specific example of the arrangement of these components will be described hereinlater.

In the present embodiment, although terminal switching portion 14 is connected to both of constant current generating portion 12 and voltage detecting portion 13 and selects both of the electrodes for the current and the electrodes for the voltage, the configuration is not limited to the above-described one. For example, instead of terminal switching portion 14, a first switching portion connected to constant current generating portion 12 for selecting only the electrodes for the current and a second switching portion connected to voltage detecting portion 13 for selecting only the electrodes for the voltage may be provided.

Further, in the present embodiment, although the electrodes for the current and the electrodes for the voltage are selected through terminal switching portion 14, the configuration may not include terminal switching portion 14. In this case, for example, a current generator may be provided for each pair of electrodes serving as the electrodes for the current and control portion 20 may control each current generator. Thus, the electrodes for the current can be switched successively without using terminal switching portion 14. Similarly, a voltage detector may be provided for each pair of electrodes serving as the electrodes for the voltage and control portion 20 may control each voltage detector. In this way, without using terminal switching portion 14, the electrodes for the voltage can be switched successively.

Furthermore, at least one of control portion 20 and drive device 23a included in upper limb unit 1 may be provided to lower limb unit 2.

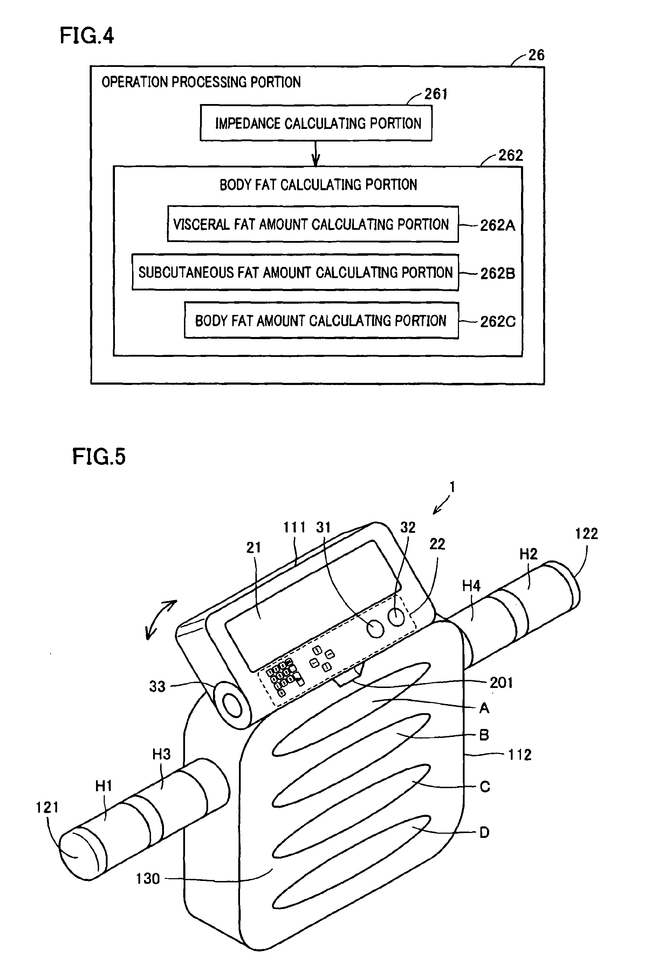

A functional configuration of operation processing portion 26 is shown in Fig. 4.

Referring to Fig. 4, in the present embodiment, operation processing portion 26 includes an impedance calculating portion 261 for calculating an impedance concerning the whole body of the subject and two types of impedances concerning the abdomen of the subject, and a body fat calculating portion 262 for calculating a body fat of the subject. In the present embodiment, the body fat includes at least an amount of visceral fat and preferably includes an amount of subcutaneous fat and an amount of body fat in addition to an amount of visceral fat. "Body fat amount" refers to an amount relevant to the body fat and includes for example at least one of the body fat weight, the body fat volume and the body fat percentage. Here, the body fat amount includes an amount of visceral fat and an amount of subcutaneous fat.

Impedance calculating portion 261 calculates each impedance based on an electric current value generated by constant current generating portion 12 and a potential difference obtained from voltage detecting portion 13 via I/F 25. One of the two types of impedances concerning the abdomen is an impedance reflecting the whole fat amount of a cross section of the abdomen of the subject (whole fat amount is namely the sum of the visceral fat amount and the subcutaneous fat amount). The other one is an impedance reflecting the subcutaneous fat amount of a cross section of the abdomen of the subject. In the following description, the impedance concerning the whole body is represented by "Zw", the impedance reflecting the whole fat amount of a cross section of the abdomen is represented by "Zt" and the impedance reflecting the subcutaneous fat amount of a cross section of the abdomen is represented by "Zs".

Body fat calculating portion 262 includes a visceral fat amount calculating portion 262A for calculating the visceral fat amount, a subcutaneous fat amount calculating portion 262B for calculating the subcutaneous fat amount and a body fat amount calculating portion 262C for calculating the body fat amount.

Visceral fat amount calculating portion 262A calculates the visceral fat amount, for example, the visceral fat area (unit: cm$^2$) of the subject based on the calculated two types of impedances Zt, Zs concerning the abdomen and the physical data (waist length) of the subject. Specifically, a relation between the two types of impedances Zt, Zs and the waist length and the visceral fat amount of the subject is represented by formula (1) as shown below which is used to calculate visceral fat area Sv.

$$Sv = a * W^2 - b * (1/Zt) - c * W * Zs - d \quad \dots (1)$$

(where a, b, c, d: factor, W: waist length)

Subcutaneous fat amount calculating portion 262B calculates the subcutaneous fat amount, for example, the subcutaneous fat area (unit: cm$^2$) of the subject based on the calculated impedance Zs concerning the abdomen and the physical data (waist length) of the subject. Specifically, for example, a relation between impedance Zs and the waist length and subcutaneous fat amount of the subject is represented by following formula (2) which is used to calculate subcutaneous fat area Ss.

$$Ss = e * W * Zs + f \quad \dots (2)$$

(where e, f: factor, W: waist length)

Body fat amount calculating portion 262C calculates the body fat amount, for example, body fat percentage (%) of the subject based on the calculated impedance Zw concerning the whole body and at least a piece of data (weight for example) included in the subject data. Specifically, the body fat percentage is calculated using following formula (3) based on fat free mass FFM and the weight of the subject.

$$\text{Body fat percentage} = (Wt - FFM) / Wt * 100 \qquad \ldots (3)$$

(where Wt: weight)

Fat free mass FFM (unit: kg) is calculated using following formula (4) representing the relation between impedance Zw and at least one piece of data (height and weight for example) included in the subject data and the fat free mass.

$$FFM = i * H^2 / Zw + j * Wt + k \qquad \ldots (4)$$

(where i, j, k: factor, H: height)

Correlation formulas (1), (2) and (4) as described above are defined by the correlation with a reference measured for example with the MRI. Such correlation formulas may be defined for each age and/or gender.

In upper limb unit 1 of body fat measuring apparatus 100, with reference to Fig. 5, display portion 21 and input portion 22 are arranged at a front surface of first housing 111 (the surface located on the same side as surface 130 of second housing 112).

Input portion 22 is placed under display portion 21 for example and includes such keys as numeric keys, determination key, back space key and cursor keys for moving a cursor on the screen upward, downward, rightward and leftward. Input portion 22 also includes a measurement start key 31 for giving an instruction to start measurement and a measurement stop key 32 for giving an instruction to stop the measurement.

Between first housing 111 and second housing 112, a hinge 33 is provided.

The position and shape of hinge 33 are not limited to particular ones. It is sufficient that first housing 111 is inclined to allow the subject to be able to confirm the results of measurement while keeping the posture for taking a measurement.

Further, preferably, an upper end portion of surface 130 (an end portion located on the head side of the subject who is in a posture for taking a measurement) is provided with a depression 201 as positioning means corresponding to the position of the navel (the positioning means is hereinafter referred as "positioning index"). More specifically, depression 201 is provided on an extension in the alignment direction of the abdomen electrodes. Thus, the subject can easily allow the abdomen electrodes to contact appropriate positions on the surface of the abdomen. Accordingly, the measurement accuracy can be improved.

In the present embodiment, the shape of surface 130 is substantially rectangular. However, the shape is not limited to this one. For example, the surface may be substantially circular.

Further, although the description is given here about main portion 110 divided into first housing 111 and second housing 112, the main portion is not limited to the form as described. Specifically, main portion 110 may be one housing, and display portion 21 and input portion 22 may be provided on the same surface as surface 130 where the abdomen electrodes are arranged.

Alternatively, the surface where display portion 21 is provided may be inclined in advance such that the subject can confirm the results of measurement while keeping the posture for taking a measurement, without hinge 33.

Alternatively, display portion 21 and/or input portion 22 may be arranged on the rear surface of main portion 110 (the surface opposite to surface 130). In this way, upper limb unit 1 can be downsized.

<Operation of Body Fat Measuring Apparatus>

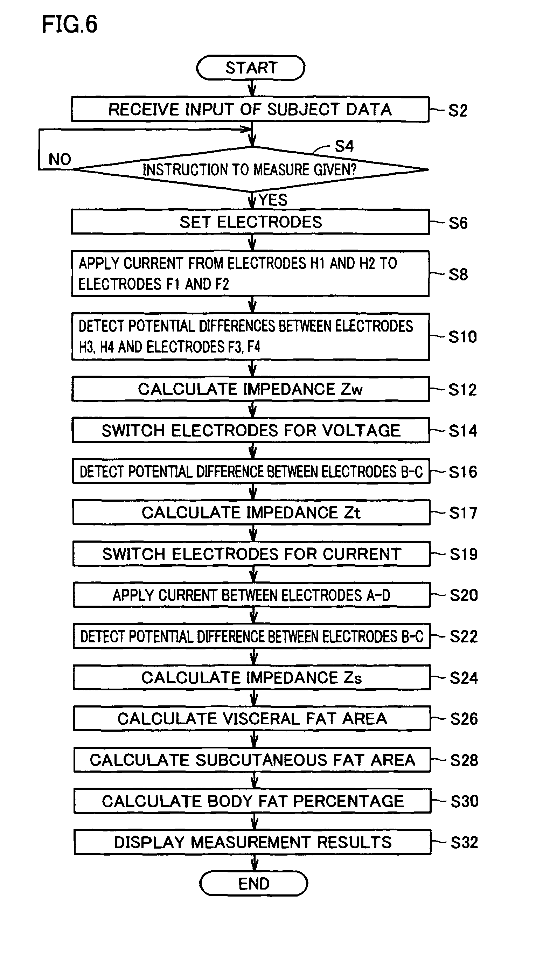

The body fat measuring process in the first embodiment of the present invention as illustrated in the flowchart of Fig. 6 is stored in advance in storage portion 27 as a program, and operation processing portion 26 reads and executes the program to implement the function of the body fat measuring process.

Referring to Fig. 6, operation processing portion 26 receives input of subject data including physical data (waist length) (step S2). The subject data received here is temporarily recorded for example in storage portion 27.

Then, operation processing portion 26 determines whether or not an instruction to start measurement is given (step S4). Operation processing portion 26 waits until the instruction to start measurement is given (NO in step S4). Detecting the instruction to start measurement (YES in S4), operation processing portion 26 sets electrodes (step S6). More specifically, operation processing portion 26 controls terminal switching portion 14 to connect two pairs of electrodes H1, H2, F1, F2 as electrodes for the current to constant current generating portion 12, and further to short-circuit electrode H1 and electrode H2 and short-circuit electrode F1 and electrode F2. Furthermore, operation processing portion 26 controls terminal switching portion 14 to connect two pairs of electrodes H3, H4, F3, F4 as the electrodes for the voltage to voltage detecting portion 13, and further to short-circuit electrode H3 and electrode H4 and short-circuit electrode F3 and electrode F4.

Subsequently, operation processing portion 26 controls constant current generating portion 12 to apply a constant current from electrodes H1 and H2 to electrodes F1 and F2 (step S8). In this state, operation processing portion 26 causes voltage detecting portion 13 to detect potential differences between electrodes H3, H4 and electrodes F3, F4 (step S10).

Impedance calculating portion 261 calculates impedance Zw based on the potential differences detected in step S10 (step S12). The value of impedance Zw calculated here is temporarily recorded for example in storage portion 27.

Then, operation processing portion 26 switches the electrodes for the voltage (step S14). More specifically, operation processing portion 26 controls terminal switching portion 14 to switch the electrodes for the voltage from electrodes H3, H4, F3, F4 to electrodes B, C. Thus, terminal switching portion 14 disconnects electrodes H3, H4, F3, F4 and voltage detecting portion 13 from each other and connects electrodes B, C and voltage detecting portion 13 to each other.

Subsequently, in the state where the constant current is applied from electrodes H1 and H2 to electrodes F1 and F2, operation processing portion 26 causes voltage detecting portion 13 to detect a potential difference between electrodes B, C (step S 16). Here, in the case where a potential difference is detected while the current is applied to the four limbs, the current may be applied for example from one of electrodes H1, H2 to one of electrodes F1, F2.

Impedance calculating portion 261 calculates impedance Zt based on the potential difference detected in step S16 (step S17). The value of impedance Zt calculated here is temporarily stored for example in storage portion 27.

Then, operation processing portion 26 switches the electrodes for the current (step S 19). More specifically, operation processing portion 26 controls terminal switching portion 14 to switch the electrodes for the current from electrodes H1, H2, F1, F2 to electrodes A, D. Thus, terminal switching portion 14 disconnects electrodes H1, H2, F1, F2 and constant current generating portion 12 from each other and connects electrodes A and D and constant current generating portion 12 to each other.

Subsequently, operation processing portion 26 controls constant current generating portion 12 to apply the current between electrodes A and D (step S20). In this state, operation processing portion 26 causes voltage detecting portion 13 to detect a potential difference between electrodes B and C (step S22). Impedance calculating portion 261 calculates impedance Zs based on the potential difference detected in step S22 (step S24). The value of impedance Zs calculated here is temporarily recorded for example in storage portion 27.

Then, visceral fat amount calculating portion 262A calculates visceral fat area Sv from the physical data (waist length) which is input in step S2 and impedance Zt and impedance Zs (step S26). Visceral fat area Sv is calculated with the above-described formula (1).

Simultaneously, subcutaneous fat amount calculating portion 262B calculates subcutaneous fat area Ss from the physical data (waist length) which is input in step S2 and impedance Zs (step S28). Subcutaneous fat area Ss is calculated with the above-described formula (2).

Further, body fat amount calculating portion 262C calculates the body fat percentage based on the subject data (height, weight) of the subject that is input in step S2 and impedances Zw (step S30). The body fat percentage is calculated with the above-described formulas (3) and (4).

Finally, operation processing portion 26 operates to display the results of measurement on display portion 21 (step S32).

After the above-described steps, the body fat measuring process in the first embodiment of the present invention is ended.

Here, impedance Zt has a typical value of approximately 5 Ω. Impedance Zs has a typical value of approximately 80 Ω. Further, impedance Zw has a typical value of approximately 250 Ω.

As described above, both of the two types of impedances Zt, Zs are calculated based on the potential difference between electrodes B and C which are disposed in the lengthwise direction of the abdomen of the subject. Therefore, the visceral fat amount can be calculated with precision. Further, since the abdomen electrodes are arranged at surface 130 of upper limb unit 1, the visceral fat amount can be easily and conveniently calculated with precision at home as well.

Although the above-described flowchart shows that the impedance is calculated each time the potential difference is detected. Alternatively, the impedances may be calculated all together after all potential differences are calculated. Further, the order in which the setting and switching of the electrodes and the detection of the potential differences are done is not limited to the above-described order.

Further, in the present embodiment, as the body fat of the subject, the visceral fat amount (visceral fat area), the subcutaneous fat amount (subcutaneous fat area) and the body fat amount (body fat percentage) are calculated. At least the visceral fat amount, however, may be calculated. In this case, in step S2, only the physical data (waist length) may be input.

Further, in the present embodiment, the body fat amount is calculated based on impedance Zw of the whole body. The body fat amount, however, may be calculated (estimated) based on impedance Zt of the abdomen. In this case, body fat measuring apparatus 100 may have the structure where electrodes for the voltage H3, H4 of the upper limb electrodes and electrodes for the voltage F3, F4 of the lower limb electrodes are not provided. Further, in the configuration, one of the electrodes for the current H1, H2 and one of the electrodes for the current F1, F2 may not be provided. In other words, body fat measuring apparatus 100 may include abdomen electrodes including at least four electrodes as well as a pair of electrodes that are one of electrodes H1 and H2 and one of electrodes F 1 and F2.

Further, upper limb unit 1 herein includes left and right grips 121, 122 formed in the shape of a handle. However, as long as the subject can hold the component with at least one hand, the form is not limited to the one as described above. In this case, at least one electrode for the current is disposed at a position held with one hand.

Further, regarding electrodes A, B, C, D included in the line of abdomen electrodes in the first embodiment described above, in both of the cases where the current is applied from four limbs and where the current is applied in the abdomen, the pair of electrodes B, C sandwiched between paired electrodes A, D is commonly used as the electrodes for the voltage. The electrodes, however, may not be used commonly to both cases. For example, in the case where the current is applied from four limbs, a pair of electrodes other than the pair of electrodes B, C may be used. For example, it is desirable in terms of accurate measurement of the value to use the pair of electrodes A, D located on the outside as the electrodes for the voltage, since the measured value is larger. Here, the pair of electrodes may be a pair of electrodes A, B or a pair of electrodes B, D

Alternatively, although the present embodiment uses, of the four electrodes A, B, C, D, the pair of electrodes A, D located on the outside as electrodes functioning as the electrodes for the current and uses the pair of electrodes B, C located on the inside as electrodes functioning as the electrodes for the voltage, the pairs of electrodes may be replaced with each other. Namely, the pair of electrodes A, D may function as the electrodes for the voltage, and the pair of electrodes B, C may function as the electrodes for the current.

Further, in the present embodiment, the impedance is calculated from the detected potential difference and the visceral fat amount for example is calculated based on the calculated impedance. The visceral fat amount for example, however, may be calculated directly from the detected potential difference. Specifically, although the above-described correlation formula (1) for example is a formula using two types of impedances $Z_t$, $Z_s$, a formula using two types of potential differences may be used.

As to examples of display:

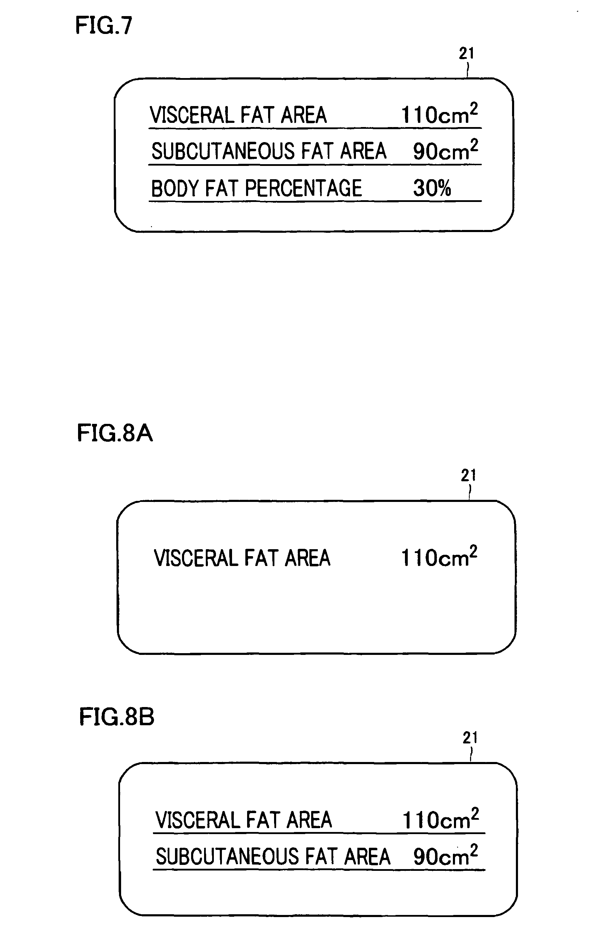

Fig. 7 illustrates an example of display of measurement results in step S32 of Fig. 6. Referring to Fig. 7, display portion 21 indicates "visceral fat area 110 $cm^2$," "subcutaneous fat area 90 $cm^2$" and "body fat percentage 30%" in respective predetermined regions where the calculated values are indicated as numerical values. Thus, the subject can know the specific numerical values of the visceral fat area, subcutaneous fat area and body fat percentage of the subject. Further, since these values are indicated simultaneously, the fat balance of the subject can further be known.

Here, the display is not limited to the example as shown in Fig. 7, and only the visceral fat area may be indicated as a result of measurement as shown in Fig. 8A, or the visceral fat area and the subcutaneous fat area may be indicated as results of measurement as shown in Fig. 8B.

The subject data may further be indicated in addition to the results of measurement.

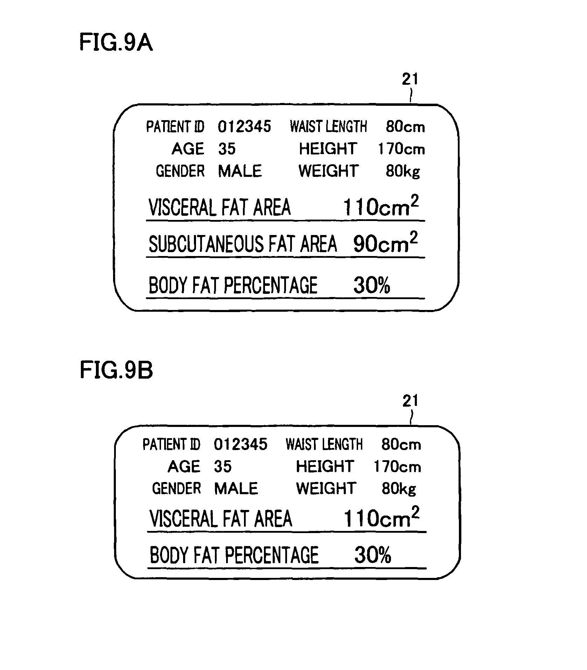

Fig. 9A shows a first diagram indicating, as subject data, the waist length which is physical data and additionally data such as patient ID, age, gender, height and weight for example. As the results of measurement, the visceral fat area, subcutaneous fat area and body fat percentage are indicated.

Fig. 9B shows a second diagram which also indicates, as subject data, the waist length which is physical data and additionally data such as patient ID, age, gender, height and weight for example. As the results of measurement, the visceral fat area and subcutaneous fat area are indicated.

When the results of measurement are indicated, a reference value or a standard value of the visceral fat area may further be indicated. "Reference value of the visceral fat area" refers to the reference value used by the Japan Society for the Study of Obesity as a criterion for making a judgment about visceral fat obesity, and specifically corresponds to 100 $cm^2$.

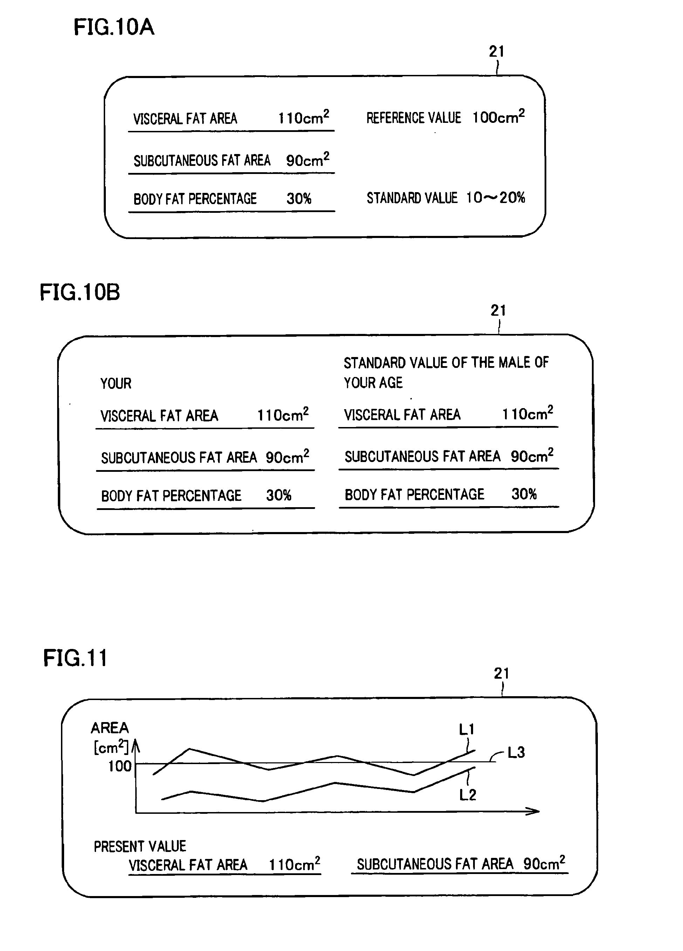

Referring to Fig. 10A, display portion 21 indicates the results of measurement similarly to Fig. 7 and additionally indicates the reference value (100 $cm^2$) of the visceral fat area at a side of the measured value (110 $cm^2$) of the visceral fat area. Thus, since the reference value of the visceral fat area is indicated together with the measured value thereof, the subject can easily know whether the subject is classified into the visceral fat obesity which is considered as the one having a high risk of lifestyle related diseases. Further as shown in Fig. 10A, at a side of the measured value (30%) of the body fat percentage, its standard value (10-20% for example) thereof may be additionally indicated.

As shown in Fig. 10B, display portion 21 indicates the results of measurement similarly to Fig. 7 and additionally indicates a standard value of those of the same age and the same gender (male/female) as the subject at a side of each result of measurement. Thus, the subject can determine whether the results of measurement of the subject are regarded as standard results.

In addition to the results of measurement, the values measured in the past may be indicated in the form of a graph. Referring to Fig. 11, display portion 21 indicates a graph showing a trend (transition) of the measured values, with the vertical axis for the area (unit: $cm^2$) and the horizontal axis for the time. In this case, the reference value (100

cm$^2$) of the visceral fat area may be indicated additionally. In this diagram, bent line L1 shows a trend of measured values of the visceral fat area in the past, and bent line L2 shows a trend of measured values of the subcutaneous fat area in the past. Straight line L3 which is parallel with the horizontal axis is a reference line indicating the reference value of the visceral fat area. Although the information about the visceral fat area and the subcutaneous fat area is displayed here, only the information about the visceral fat area may be displayed. Further, the graph of bent line L1 concerning the visceral fat area and the graph of bent line L2 concerning the subcutaneous fat area may be indicated separately.

Although the above-described first embodiment provides depression 201 as a positioning index in main portion 110, the index is not limited to this.

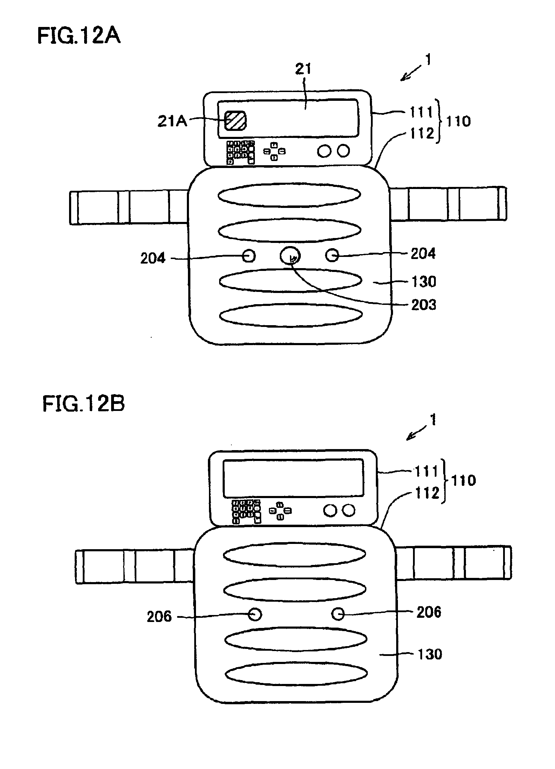

As shown in Fig. 12A, a camera 203 that can shoot an image may be provided as a positioning index at surface 130. In this drawing, camera 203 is placed for example between electrode B and electrode C and on a line connecting the central point of electrode B and the central point of electrode C.

In this case, for example, in a predetermined region 21A of display portion 21, an image shot by camera 203 is displayed. Thus, the subject can allow surface 130 to contact the surface of the abdomen while seeing the navel of the subject.

Further, preferably a lighting apparatus such as light 204 is provided near camera 203 at surface 130. Thus, the disadvantage that the position of the navel cannot be seen because of the darkness when surface 130 is drawn near the abdomen can be prevented.

Here, an image display portion for displaying the image shot by camera 203 may be provided in a region separate from display portion 21.

Alternatively, as shown in Fig. 12B, as still another example of the positioning index, an irradiation apparatus emitting a laser beam for directly irradiating the position of the navel, for example, a laser LED (Light Emitting Diode) 206 may be placed at surface 130. In this drawing, two laser LEDs 206 are provided between electrode B and electrode C and in substantially parallel with the alignment direction of the abdomen electrodes. Here, the position where laser LED 206 is provided is not limited to the above-described one.

Upper limb unit 1 of the above-described first embodiment includes connector 16a therein and thus is replaceable with an upper limb unit of any body fat meter (a body fat meter like body fat measuring apparatus 100 that is formed of an upper limb unit, a lower limb unit and a cable for electrically connecting these units) already available in the market. Therefore, a subject who has already owned such a body fat meter can measure the visceral fat amount more accurately than before by merely purchasing upper limb unit 1.

[Second Embodiment]

Regarding body fat measuring apparatus 100 in the first embodiment, only a single line of abdomen electrodes is arranged at surface 130 of upper limb unit 1. In a second embodiment, multiple lines of abdomen electrodes are arranged at the upper limb unit. In the following, main differences between the second embodiment and the first embodiment will be described.

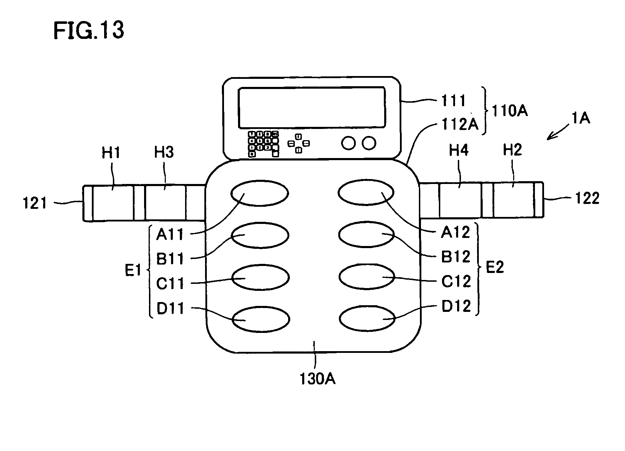

Referring to Fig. 13, an upper limb unit 1A of a body fat measuring apparatus 100 in the second embodiment of the present invention includes two lines of abdomen electrodes E1, E2 at a surface 130A of a second housing 112A that is a component of a main portion 110A. Abdomen electrode E1 includes four electrodes A11,B 11, C11, D11, and abdomen electrode E2 includes four electrodes A12, B12, C12, D12. These two lines of abdomen electrodes E1, E2 are spaced apart from each other by a predetermined distance. Further, it is desirable that the electrodes that are components of abdomen electrodes E1, E2 are arranged in a matrix.

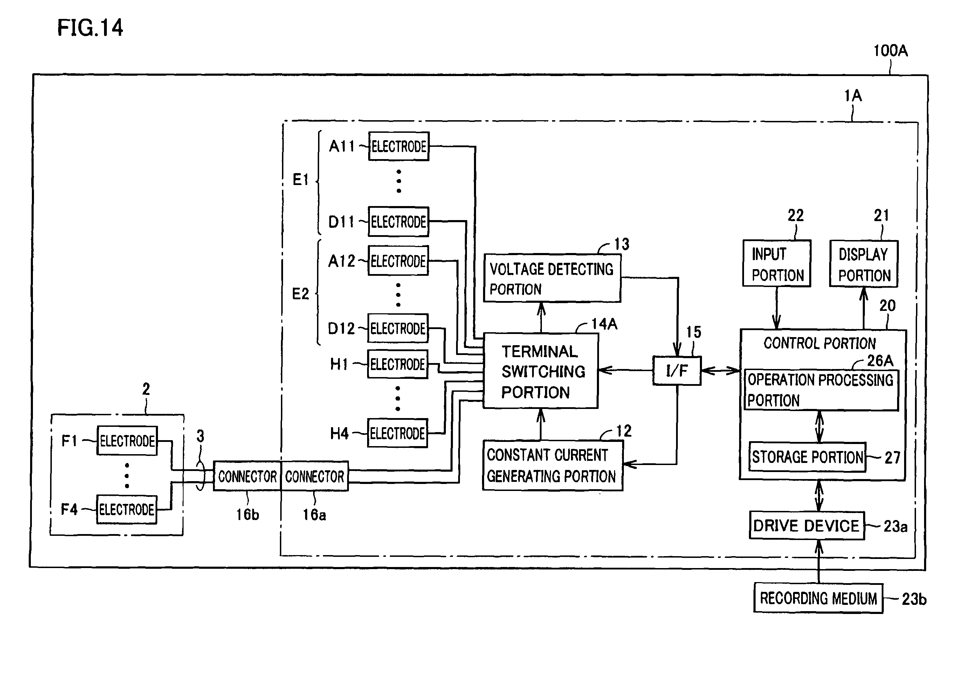

Referring to Fig. 14, upper limb unit 1A of body fat measuring apparatus 100 in the second embodiment of the present invention is provided with electrodes A11, ... , D11 (abdomen electrode E1) and electrodes A12, ... , D12 (abdomen electrode E2) instead of electrodes A, ... , D shown in Fig. 3. Accordingly, each electrode of abdomen electrodes E1, E2 and a terminal switching portion 14A are connected.

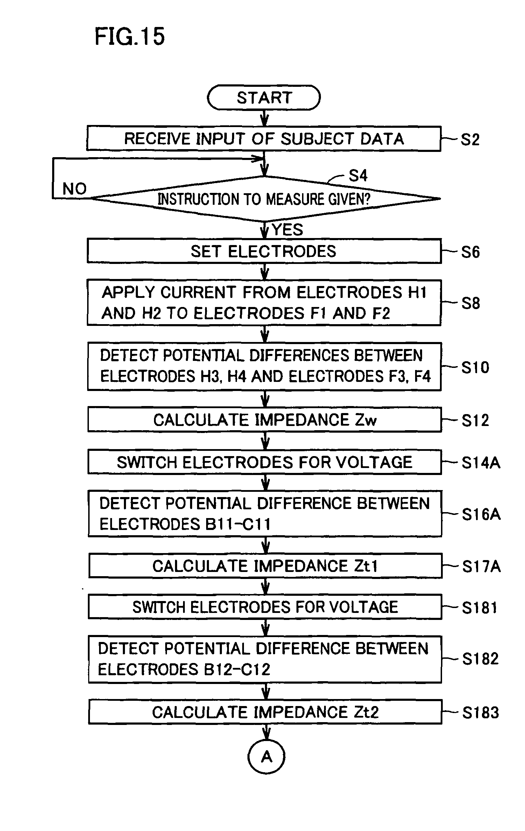

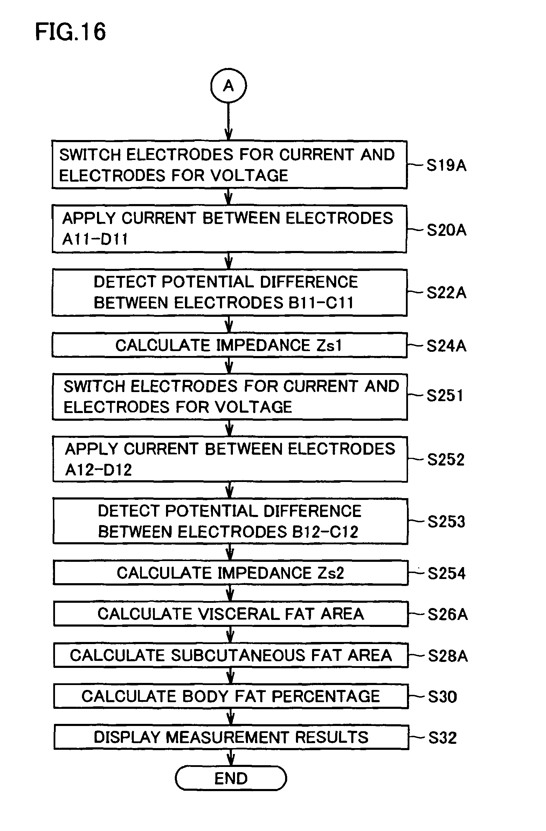

A body fat measuring process in the second embodiment of the present invention as shown in the flowcharts of Figs. 15 and 16 is stored as a program in storage portion 27 in advance. An operation processing portion 26A reads and executes this program to implement the function of the body fat measuring process. Here, any process step similar to a corresponding step in the body fat measuring process of the first embodiment is indicated by the same step number.

Referring to Fig. 15, operation processing portion 26A switches electrodes for the voltage, after respective operations in steps S2 to S12, (step S 14A). More specifically, operation processing portion 26A controls terminal switching portion 14A to switch the electrodes for the voltage from electrodes H3, H4, F3, F4 to electrodes B11, C11. Thus, terminal switching portion 14A disconnects electrodes H3, H4, F3, F4 and voltage detecting portion 13 from each other and connects electrodes B11, C11 and voltage detecting portion 13 to each other.

Subsequently, in the state where a constant current is applied from electrode H1 and electrode H2 to electrode F 1 and electrode F2, operation processing portion 26A causes voltage detecting portion 13 to detect a potential difference between electrodes B11, C11 (step S16A).

Impedance calculating portion 261 calculates impedance Zt1 based on the potential difference detected in step S 16A (step S17A). The value of impedance Zt 1 calculated here is temporarily stored for example in storage portion 27.

Further, operation processing portion 26A switches the electrodes for the voltage (step S181). More specifically, operation processing portion 26A controls terminal switching portion 14A to switch the electrodes for the voltage from electrodes B11, C11 to electrodes B12, C12. Thus, terminal switching portion 14A disconnects electrodes B11, C 11 and voltage detecting portion 13 from each other and connects electrodes B12, C12 and voltage detecting portion 13 to each other.

Subsequently, in the state where a constant current is applied from electrode H1 and electrode H2 to electrode F 1 and electrode F2, operation processing portion 26A causes voltage detecting portion 13 to detect a potential difference between electrodes B12, C12 (step S 182).

Impedance calculating portion 261 calculates impedance Zt2 based on the potential difference detected in step S182 (step S183). The value of impedance Zt2 calculated here is temporarily stored in storage portion 27 for example.

Referring next to Fig. 16, operation processing portion 26A switches the electrodes for the current and the electrodes for the voltage (step S 19A). More specifically, operation processing portion 26A controls terminal switching portion 14A to switch the electrodes for the current from electrodes H1, H2, F1, F2 to electrodes A11, D11. Thus, terminal switching portion 14A disconnects electrodes H1, H2, F1, F2 and constant current generating portion 12 from each other and connects electrode A11 and electrode D11 and constant current generating portion 12 to each other. Further, operation processing portion 26A controls terminal switching portion 14A to switch the electrodes for the voltage from electrodes B12, C12 to electrodes B11, C11. Thus, terminal switching portion 14A disconnects electrode B12, C12 and voltage detecting portion 13 from each other and connects electrodes B11, C11 and voltage detecting portion 13 to each other.

Subsequently, operation processing portion 26A controls constant current generating portion 12 to apply a current between electrode A11 and electrode D11 (step S20A). In this state, operation processing portion 26A causes voltage detecting portion 13 to detect a potential difference between electrode B11 and electrode C 11 (step S22A). Impedance calculating portion 261 calculates impedance Zs1 based on the potential difference detected in step 22A (step S24A). The value of impedance Zs1 calculated here is temporarily stored for example in storage portion 27.

Further, operation processing portion 26A switches the electrodes for the current and the electrodes for the voltage (step S251). More specifically, operation processing portion 26A controls terminal switching portion 14A to switch the electrodes for the current from electrodes A11, D11 to electrodes A12, D12. Thus, terminal switching portion 14A disconnects electrode A11 and electrode D11 and constant current generating portion 12 from each other and connects electrode A12 and electrode D12 and constant current generating portion 12 to each other. Further, operation processing portion 26A controls terminal switching portion 14A to switch the electrodes for the voltage from electrodes B11, C11 to electrodes B12, C 12. Thus, terminal switching portion 14A disconnects electrodes B11, C11 and voltage detecting portion 13 from each other and connects electrodes B12, C12 and voltage detecting portion 13 to each other.

Subsequently, operation processing portion 26A controls constant current generating portion 12 to apply a current between electrode A12 and electrode D12 (step S252). In this state, operation processing portion 26A causes voltage detecting portion 13 to detect a potential difference between electrode B12 and electrode C12 (step S253). Impedance calculating portion 261 calculates impedance Zs2 based on the potential difference detected in step S253 (step S254). The value of impedance Zs2 calculated here is temporarily stored for example in storage portion 27.

After the operation in step S254, visceral fat amount calculating portion 262A calculates visceral fat area Sv from the physical data (waist length) which is input in step S2 and impedances Zt1, Zt2 and impedances Zs1, Zs2 (step S26A). Visceral fat area Sv is calculated with the above-described formula (1). In the case where two lines of abdomen electrodes E1, E2 are provided like those in the second embodiment, the average value of two impedances Zt1, Zt2 may be substituted for impedance Zt in correlation formula (1) and the average of two impedances Zs1, Zs2 may be substituted for impedance Zs in correlation formula (1).

Simultaneously, subcutaneous fat amount calculating portion 262B calculates subcutaneous fat area Ss from the physical data (waist length) which is input in step S2 and impedances Zs1, Zs2 (step S28A). Subcutaneous fat area Ss is calculated with the above-described formula (2). In this case as well, the average of two impedances Zs1, Zs2 may be substituted for impedance Zs in correlation formula (2).

After the operation in step S28A, the body fat calculating operation (step S30) and the measurement results display operation (step S32) similar to those in the first embodiments are performed. After the above-described steps, the body fat measuring process in the second embodiment of the present invention is ended.

Thus, a plurality of abdomen electrodes E1, E2 are provided and a plurality of potential differences are averaged, so that influences of the fat distribution and the fat thickness can be reduced.

Here, when the body fat is calculated, impedances Zt1, Zt2 and impedances Zs1, Zs2 are averaged. The method is not limited to the above-described one. Based on an average value of a plurality of potential differences detected by applying the current from the four limbs, impedance Zt may be calculated and, based on an average of a plurality of potential differences detected by applying the current from the abdomen, impedance Zs may be calculated.

Further, although impedances Zt1, Zt2, and impedances Zs1, Zs2 are averaged for using the averages in calculating the visceral fat amount and subcutaneous fat amount, any operation other than the averaging may be performed. For example, a correlation formula may be provided for each impedance to calculate the visceral fat amount or subcutaneous fat amount.

It is preferable that upper limb unit 1A in the second embodiment is also provided with a positioning index in terms of improvement of the measurement accuracy.

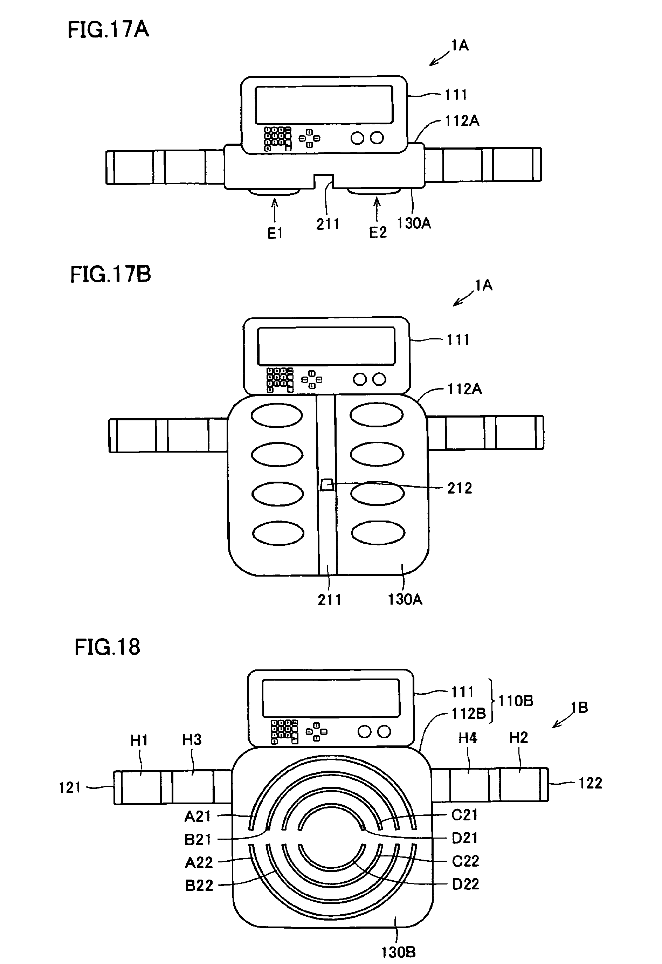

Referring to Fig. 17A, a groove 211 for example may be provided as a positioning index in surface 130A of upper limb unit 1A. Groove 211 is provided between abdomen electrodes E1, E2 and in parallel with the alignment direction of abdomen electrodes E1, E2, from the upper end portion to the lower end portion of second housing 112A. Groove 211 may not necessarily be provided from the upper end portion to the lower end portion of second housing 112A. Like depression 201 in the first embodiment, the groove may be provided at least in the upper end portion.

Referring to Fig. 17B, a mirror 212 for example may be provided as another example of the positioning index at surface 130A of upper limb unit 1A. Mirror 212 is provided between a line connecting electrode B 11 and electrode B 12 and a line connecting electrode C11 and electrode C12. Further, mirror 212 is provided in groove 211. Preferably mirror 212 is inclined so that the subject who holds grips 121, 122 and assumes the posture for taking a measurement can visually identify the navel of the subject. Specifically, mirror 212 is preferably inclined so that an incoming image which is perpendicular to surface 130A is reflected in the direction toward the first housing 111 and then in the direction changed toward the incoming side. Mirror 212 may be embedded in second housing 112A.

Thus, groove 211 or mirror 212 is provided to upper limb unit 1A so that the subject taking the measurement can allow surface 130A to contact the surface of the abdomen while seeing the navel of the subject from the upper surface side of upper limb unit 1A.

[Third Embodiment]

Regarding body fat measuring apparatus 100 in the first embodiment, a single line of abdomen electrodes includes four electrodes (two pairs of electrodes) at surface 130 of upper limb unit 1. In a third embodiment, abdomen electrodes include three or more pairs of electrodes or five or more electrodes. In the following, main differences between the third embodiment and the first embodiment will be described.

Referring to Fig. 18, an upper limb unit 1B of a body fat measuring apparatus 100B in the third embodiment of the present invention includes, at a surface 130B of a second housing 112B that is a component of a main portion 110B, substantially arc-shaped electrodes A21, B21, C21, D21, D22, C22, B22, A22 arranged in this order from the upper side. Electrodes A21, B21, C21, D21 are arranged such that each electrode is curved toward the upper end of surface 130B. Electrodes D22, C22, B22, A22 are arranged such that each electrode is curved toward the lower end of surface 130B. More specifically, a pair of electrodes B21, B22 is arranged inside a pair of electrodes A21, A22, and a pair of electrodes C21, C22 is arranged inside the pair of electrodes B21, B22. Further, a pair of electrodes D21, D22 is arranged inside the pair of electrodes C21, C22.

Here, four pairs of arc-shaped electrodes that are different in size (radius or diameter) are provided. The electrodes are not limited to the above-described ones. For example, three or more pairs or five or more pairs of arc-shaped electrodes different from each other in size may be provided. Alternatively, multiple pairs of arc-shaped electrodes having the same size may be provided. In the case where two pairs of arc-shaped electrodes are provided, a process similar to the above-described one in the first embodiment can be performed.

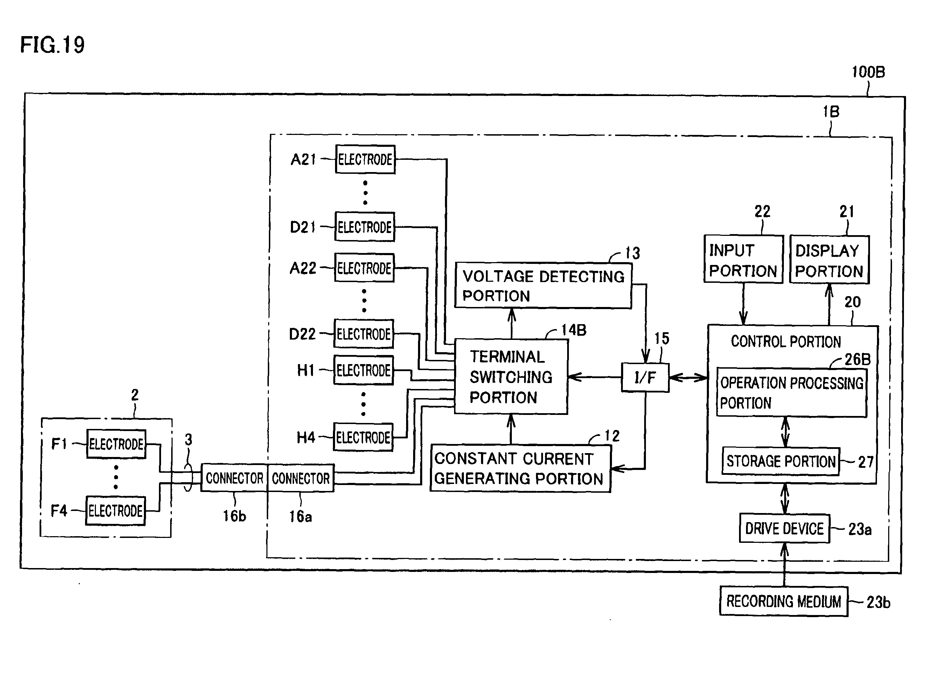

Referring to Fig. 19, upper limb unit 1B of body fat measuring apparatus 100B in the third embodiment of the present invention is provided with electrodes A21, ... , D21 and electrodes A22, ... , D22 instead of electrodes A, ... , D shown in Fig. 3. Accordingly, electrodes that are component of the abdomen electrodes are each connected to a terminal switching portion 14B.

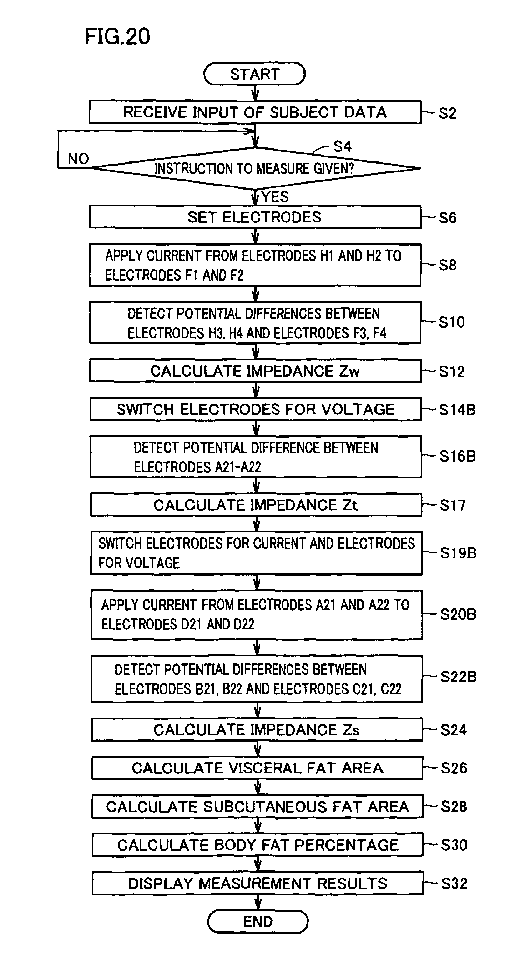

A body fat measuring process in the third embodiment of the present invention as shown in the flowchart of Fig. 20 is stored as a program in storage portion 27 in advance. An operation processing portion 26B reads and executes the program to implement the function of the body fat measuring process. Any step similar to a corresponding step of the body fat measuring process in the first embodiment is indicated by the same step number.

Referring to Fig. 20, operation processing portion 26B switches the electrodes for the voltage, after respective operations in steps S2 to S 12 (step S 14B). More specifically, operation processing portion 26B controls terminal switching portion 14B to switch the electrodes for the voltage from electrodes H3, H4, F3, F4 to electrodes A21, A22. Thus, terminal switching portion 14B disconnects electrodes H3, H4, F3, F4 and voltage detecting portion 13 from each other and connects electrodes A21, A22 and voltage detecting portion 13 to each other.

Subsequently, in the state where a constant current is applied from electrode H1 and electrode H2 to electrode F1 and electrode F2, operation processing portion 26B causes voltage detecting portion 13 to detect a potential difference between electrodes A21, A22 (step S16B). Impedance calculating portion 261 calculates impedance Zt based on the potential difference detected in step S 16B (step S 17).

Then, operation processing portion 26B switches the electrodes for the current and the electrodes for the voltage (step S19B). More specifically, operation processing portion 26B controls terminal switching portion 14B to switch the electrodes for the current from electrodes H1, H2, F1, F2 to electrodes A21, D21, A22, D22. Thus, terminal switching portion 14B disconnects electrodes H1, H2, F1, F2 and constant current generating portion 12 from each other and connects electrodes A21, D21, A22, D22 and constant current generating portion 12 to each other. Further, operation processing portion 26B controls terminal switching portion 14B to switch the electrodes for the voltage from electrodes A21, A22 to electrodes B21, C21, B22, C22. Thus, terminal switching portion 14B disconnects electrodes A21, A22 and voltage detecting portion 13 from each other and connects electrodes B21, C21, B22, C22 and voltage detecting portion 13 to each other. In this case, operation processing portion 26B short-circuits electrode A21 and electrode A22 and short-circuits electrode D21 and electrode D22. Likewise, operation processing portion 26B short-circuits electrode B21 and electrode B22 and short-circuits electrode C21 and electrode C22.

Subsequently, operation processing portion 26B controls constant current generating portion 12 to apply a current from electrode A21 and electrode A22 to electrode D21 and electrode D22 (step S20B). In this state, operation processing portion 26B causes voltage detecting portion 13 to detect potential differences between electrodes B21, B22 and electrodes C21, C22 (step S22B).

After the operation in step S22B, respective operations in steps S24 to S32 are performed as those in the first embodiment. After the above-described steps, the body fat measuring process in the third embodiment of the present invention is ended.

Thus, substantially arc-shaped electrodes are provided to measure the impedance of the whole surface of the abdomen with its center located at the position of the navel of the abdomen, and therefore the distance between the electrodes and the area of the electrodes can be increased and accordingly the impedance can be stably measured with higher precision.

In the above-described flowchart, the electrodes used for applying the current and the electrodes used for detecting the voltage selected from the abdomen electrodes are shown as an example and the electrodes to be selected are not limited to the above-described electrodes.

For example, while the electrodes used for detecting the voltage in the state where the current is applied to the four limbs are electrodes A21, A22 of one pair located on the outermost side, another pair of electrodes may be used instead. Alternatively, two pairs of electrodes of the abdomen electrodes may be used as electrodes for detecting the voltage. In this case, like step S22B, the voltage may be detected in the state where two pairs of electrodes are short-circuited, or potential differences between the two pairs of electrodes may be detected without short-circuiting electrodes. In the case where respective potential differences between two pairs of electrodes are detected, the two potential differences as detected may be averaged for calculating impedance Zt.

Alternatively, in the case where the current is applied to the abdomen, the curent may simply be applied for example from electrode A21 to electrode A22. Further, while the two pairs of electrodes used for detecting the voltage in the state where the current is applied to the abdomen are short-circuited, respective potential differences may be detected without short-circuiting electrodes. In this case as well, the two potential differences as detected may be averaged for calculating impedance Zs.

Further, the abdomen electrodes in the third embodiment may be provided in two lines to be combined with the body fat measuring process in the second embodiment.

It is preferable that upper limb unit 1B in the third embodiment is also provided with a positioning index in terms of enhancement of the measurement accuracy.

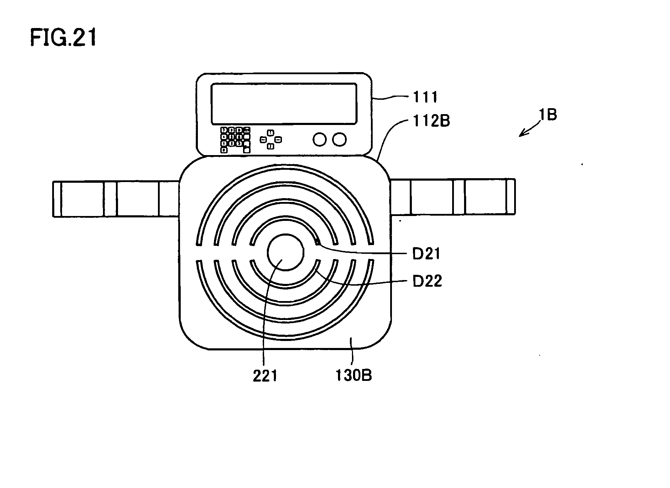

As an example of the positioning index in the third embodiment of the present invention, referring to Fig. 21, second housing 112B of upper limb unit 1B is provided with a window 221, as a positioning index, allowing visual identification from the rear side to the front side (surface 130B) for example. Window 221 is provided at a position surrounded by electrodes D21, D22 located on the innermost side (near the central portion). Thus, the visual identification can be made from the rear side to the front side, so that a helper (person helping the subject in the measurement) can see the navel of the subject through window 221. Therefore, window 221 can be formed at a position corresponding to the navel to allow abdomen electrodes to be easily arranged at appropriate positions of the surface of the abdomen of the subject.

Although window 221 is provided here as a positioning index, the window may be any that allows visual identification from the rear side to the front side of second housing 112B. Therefore, instead of window 221, a hole (not shown) passing through from the front side to the rear side may be provided.

Further, the positioning index (depression for example) illustrated in the first embodiment or the positioning index (mirror for example) illustrated in the second embodiment may be provided to second housing 112B.

Likewise, the positioning index illustrated in the second or third embodiment may be provided to second housing 112 in the first embodiment. Alternatively, the positioning index illustrated in the first or third embodiment may be provided to second housing 112A in the second embodiment.

A body fat measuring method performed by the body fat measuring apparatus or upper limb unit of the present invention may be provided as a program. Such a program may be provided as a program product recorded on an optical medium such as CD-ROM (Compact Disk-ROM) or a computer-readable recording medium such as memory card. The program may also be provided as the one downloaded via a network.

The provided program product is installed in a program storage unit such as hard disk and the installed program is executed. The program product here includes the program itself and the recording medium on which the program is recorded.

It should be construed that embodiments disclosed above are by way of illustration in all respects, not by way of limitation. It is intended that the scope of the present invention is defined by claims, not by the embodiments and examples above, and includes all modifications and variations equivalent in meaning and scope to the claims.

**Claims**

1. A body fat measuring apparatus comprising:

   an upper limb unit (1) including a hold portion (121, 122) which is adapted to be held in a hand of a subject, a display portion (21) for displaying a visceral fat amount, an input portion (22) for entering physical data of said subject and a main portion (110),
   said hold portion (121, 122) including an upper limb electrode (H1, H2, H3, H4) which is adapted to be contacted with a palm part of said subject,
   said main portion (110) including
   a first surface (130) which is adapted to be contacted with a surface of an abdomen of said subject, and
   a group of abdomen electrodes (A, B, C, D) which are arranged at said first surface (130), and
   said group of abdomen electrodes (A, B, C, D) including a pair of first electrodes (B, C) and a pair of second electrodes (A, D),
   said body fat measuring apparatus further comprising:
   a lower limb electrode (F1, F2, F3, F4) which is adapted to be contacted with a lower limb of said subject;
   an applying portion (12, 14) for applying an electric current to said subject via a pair of electrodes consisting of said upper limb electrode (H1, H2, H3, H4) and said lower limb electrode (F1, F2, F3, F4) and via the pair of said second electrodes (A, D);
   a detecting portion (13) for detecting two types of potential differences respectively in a first case where the electric current is applied via the pair of said upper limb electrode (H1, H2, H3, H4) and said lower limb electrode (F1, F2, F3, F4), and in a second case where the electric current is applied via the pair of said second electrodes (A, D),
   said detecting portion (13) detecting a first potential difference between electrodes of a predetermined pair of said first or second electrodes in said first case, and a second potential difference between said first electrodes (A, D) in said second case; and
   a visceral fat amount calculating portion (262A) for calculating a visceral fat amount of said subject based on said first potential difference and said second potential difference that are detected and physical data of said subject.
   said display portion (21) being adapted to dispaly said calculated visceral fat amount.

2. The body fat measuring apparatus according to claim 1,
   wherein
   said first electrodes (B, C) and said second electrodes (A, D) are arranged at said first surface (130) in an alignment direction that is a vertical direction in a state where said subject takes a posture for taking a measurement and holds said hold portion (121, 122).

3. The body fat measuring apparatus according to claim 2,
   wherein
   said second electrodes (A, D) are arranged at respective positions such that said first electrodes (B, C) are arranged between said second electrodes (A, D) in said alignment direction.

4. The body fat measuring apparatus according to claim 2,
   wherein
   the electrodes of said predetermined pair are said first electrodes (B, C).

5. The body fat measuring apparatus according to claim 2,

wherein
the electrodes of said predetermined pair are a pair of electrodes other than the pair of said first electrodes (B, C) included in said group of abdomen electrodes.

6. The body fat measuring apparatus according to claim 5,
wherein
said second electrodes (A, D) are arranged at respective positions such that said first electrodes (B, C) are arranged between said second electrodes (A, D) in said alignment direction, and
the electrodes of said predetermined pair are said second electrodes (A, D).

7. The body fat measuring apparatus according to claim 1, further comprising an impedance calculating portion (261) for calculating two types of impedances based on said first potential difference and said second potential difference respectively, wherein
said visceral fat amount calculating portion (262A) calculates said visceral fat amount based on said two types of impedances that are calculated and said physical data of said subject.

8. The body fat measuring apparatus according to claim 7,
wherein
said visceral fat amount calculating portion (262A) calculates said visceral fat amount using a predetermined correlation formula of a relation between said two types of impedances, said physical data and the visceral fat amount.

9. The body fat measuring apparatus according to claim 1, further comprising a subcutaneous fat amount calculating portion (262B) for calculating a subcutaneous fat amount of said subject based on said second potential difference as detected and said physical data of said subject.

10. The body fat measuring apparatus according to claim 1,
wherein
said physical data includes at least one of circumference of the abdomen, lateral width of the abdomen, thickness of the abdomen, height, and weight of said subject.

11. The body fat measuring apparatus according to claim 1, further comprising a lower limb unit (2) which is adapted such that a foot of said subject can be placed thereon, wherein
said lower limb electrode (F1, F2, F3, F4) is provided at said lower limb unit (2).

12. The body fat measuring apparatus according to claim 1,
wherein
said main portion (110) further includes positioning means (201, 203, 206, 211, 212, 221) which is adapted to be arranged at a position corresponding to a navel position of said subject.

13. The body fat measuring apparatus according to claim 12,
wherein
said positioning means is formed by a depression (201) provided in an upper end portion of said first surface (130).

14. The body fat measuring apparatus according to claim 12,
wherein
said positioning means includes
a camera (203) provided at said first surface (130), and
wherein the display portion (21) comprises a predetermined region (21A) for displaying an object whose image is shot by said camera (203).

15. The body fat measuring apparatus according to claim 12,
wherein
said positioning means is formed by a mirror (212) provided at said first surface (130).

16. The body fat measuring apparatus according to claim 12,
wherein
said positioning means is formed by an irradiating apparatus (206) provided at said first surface (130) for applying laser light.

17. The body fat measuring apparatus according to claim 12,
wherein
said main portion (110) further includes a second surface (130B) opposite to said first surface (130), and
said positioning means is formed by a hole (221) passing through from said first surface (130) toward said second surface (130).

18. The body fat measuring apparatus according to claim 12,
wherein
said main portion (110) further includes a second surface (130B) opposite to said first surface (130), and
said positioning means is formed by a window (221) providing visibility from said second surface (130B) toward said first surface (130).

**Patentansprüche**

1. Körperfettmessvorrichtung, aufweisend:

eine Einheit (1) für eine obere Extremität mit einem Halteabschnitt (121, 122), der eingerichtet ist, in einer Hand eines Subjekts gehalten zu werden, einem Anzeigeabschnitt (21) zum Anzeigen eines Viszeralfettbetrags, einem Eingabeabschnitt (22) zum Eingeben von physischen Daten des Subjekts und einem Hauptabschnitt (110),
wobei der Halteabschnitt (121, 122) eine Elektrode (H1, H2, H3, H4) für eine obere Extremität aufweist, welche eingerichtet ist, mit einem Handflächenteil des Subjekts in Berührung zu treten,
wobei der Hauptabschnitt (110) aufweist
eine erste Fläche (130), welche eingerichtet ist, mit einer Fläche eines Abdomens des Subjekts in Berührung zu treten, und
eine Gruppe Abdomenelektroden (A, B, C, D), welche an der ersten Fläche (130) angeordnet ist,
wobei die Gruppe Abdomenelektroden (A, B, C, D) ein Paar erster Elektroden (B, C) und ein Paar zweiter Elektroden (A, D) aufweist, wobei die Körperfettmessvorrichtung weiterhin aufweist:

eine Elektrode (F1, F2, F3, F4) für eine untere Extremität, welche eingerichtet ist, mit einer unteren Extremität des Subjekts in Berührung zu treten;
einen Beaufschlagungsabschnitt (12, 14) zur Beaufschlagung eines elektrischen Stroms auf das Subjekt über ein Paar Elektroden, das aus der Elektrode (H1, H2, H3, H4) für die obere Extremität und der Elektrode (F1, F2, F3, F4) für die untere Extremität besteht, und über das Paar zweiter Elektroden (A, D);
einen Erfassungsabschnitt (13) zum Erfassen von zwei Arten von entsprechenden Potentialdifferenzen, nämlich in einem ersten Fall, bei dem der elektrische Strom über das Paar aus der Elektrode (H1, H2, H3, H4) für die obere Extremität und der Elektrode (F1, F2, F3, F4) für die untere Extremität aufgebracht wird, und in einem zweiten Fall, bei dem der elektrische Strom über das Paar zweiter Elektroden (A, D) aufgebracht wird,
wobei der Erfassungsabschnitt (13) eine erste Potentialdifferenz zwischen Elektroden eines vorbestimmten Paars aus den ersten oder zweiten Elektroden in dem ersten Fall und eine zweite Potentialdifferenz zwischen den ersten Elektroden (A, D) in dem zweiten Fall erfasst; und
einen Viszeralfettbetragberechnungsabschnitt (262A) zum Berechnen eines Viszeralfettbetrags des Subjekts auf der Grundlage der erfassten ersten Potentialdifferenz und der erfassten zweiten Potentialdifferenz und der physischen Daten des Subjekts, wobei der Anzeigeabschnitt (21) eingerichtet ist, den berechneten Viszeralfettbetrag anzuzeigen.

2. Körperfettmessvorrichtung gemäß Anspruch 1, wobei die ersten Elektroden (B, C) und die zweiten Elektroden (A, D) an der ersten Fläche (130) in einer Ausrichtungsrichtung angeordnet sind, die in einer vertikalen Richtung in einem Zustand ist, bei dem das Subjekt eine Messhaltung einnimmt und den Halteabschnitt (121, 122) hält.

3. Körperfettmessvorrichtung gemäß Anspruch 2, wobei die zweiten Elektroden (A, D) an entsprechenden Positionen angeordnet sind, so dass die ersten Elektroden (B, C) zwischen den zweiten Elektroden (A, D) in der Ausrichtungsrichtung angeordnet sind.

4. Körperfettmessvorrichtung gemäß Anspruch 2, wobei die Elektroden des vorbestimmten Paars die ersten Elektroden (B, C) sind.

5. Körperfettmessvorrichtung gemäß Anspruch 2, wobei die Elektroden des vorbestimmten Paars ein Paar anderer Elektroden als das Paar erster Elektroden (B, C), die in der Gruppe Abdomenelektroden umfasst sind, sind.

6. Körperfettmessvorrichtung gemäß Anspruch 5, wobei die zweiten Elektroden (A, D) an entsprechenden Positionen angeordnet sind, so dass die ersten Elektroden (B, C) zwischen den zweiten Elektroden (A, D) in der Ausrichtungsrichtung angeordnet sind, und
wobei die Elektroden des vorbestimmten Paars die zweiten Elektroden (A, D) sind.

7. Körperfettmessvorrichtung gemäß Anspruch 1, weiterhin aufweisend einen Impedanzberechnungsabschnitt (261) zum Berechnen von zwei Arten von jeweiligen Impedanzen auf der Grundlage der ersten Potentialdifferenz und der zweiten Potentialdifferenz, wobei
der Viszeralfettbetragberechnungsabschnitt (262A) den Viszeralfettbetrag auf der Grundlage der zwei Arten von berechneten Impedanzen und der physischen Daten des Subjekts berechnet.

8. Körperfettmessvorrichtung gemäß Anspruch 7, wobei der Viszeralfettbetragberechnungsabschnitt (262A) den Viszeralfettbetrag unter Verwendung einer vorbestimmten Korrelationsformel aus einer Beziehung zwischen den zwei Arten von Impedanzen, den physischen Daten und dem Viszeralfettbetrag berechnet.

9. Körperfettmessvorrichtung gemäß Anspruch 1, weiterhin aufweisend einen Subkutanfettbetragberechnungsabschnitt (262B) zum Berechnen eines Subkutanfettbetrags des Subjekts auf der Grundlage der erfassten zweiten Potentialdifferenz und der physischen Daten des Subjekts.

10. Körperfettmessvorrichtung gemäß Anspruch 1, wobei die physischen Daten zumindest ein Element aus einem Abdomenumfang, einer lateralen Breite des Abdomens, der Dicke des Abdomens, der Größe und des Gewichts des Subjekts umfassen.

11. Körperfettmessvorrichtung gemäß Anspruch 1, weiterhin aufweisend eine Einheit (2) für eine untere Extremität, welche derart eingerichtet ist, dass ein Fuß des Subjekts darauf platziert werden kann, wobei die Elektrode (F1, F2, F3, F4) für die untere Extremität an der Einheit (2) für die untere Extremität vorgesehen ist.

12. Körperfettmessvorrichtung gemäß Anspruch 1, wobei der Hauptabschnitt (110) weiterhin Positioniermittel (201, 203, 206, 211, 212, 221) umfasst, welche eingerichtet sind, an einer Position entsprechend einer Bauchnabelposition des Subjekts angeordnet zu werden.

13. Körperfettmessvorrichtung gemäß Anspruch 12, wobei die Positioniermittel durch eine Vertiefung (201) ausgebildet werden, die in einem oberen Endabschnitt der ersten Fläche (130) vorgesehen ist.

14. Körperfettmessvorrichtung gemäß Anspruch 12, wobei die Positioniermittel umfassen
eine Kamera (203), die an der ersten Fläche (130) vorgesehen ist, und
wobei der Anzeigeabschnitt (21) einen vorbestimmen Bereich (21A) zur Anzeige eines Objekts aufweist, dessen Bild durch die Kamera (203) aufgenommen wird.

15. Körperfettmessvorrichtung gemäß Anspruch 12, wobei die Positioniermittel durch einen Spiegel (212) ausgebildet werden, der an der ersten Fläche (130) vorgesehen ist.

16. Körperfettmessvorrichtung gemäß Anspruch 12, wobei die Positioniermittel durch eine Bestrahlungsvorrichtung (206), die an der ersten Fläche (130) vorgesehen ist, zur Beaufschlagung eines Laserlichts ausgebildet sind.

17. Körperfettmessvorrichtung gemäß Anspruch 12, wobei
der Hauptabschnitt (110) weiterhin eine zweite Fläche (130B) aufweist, die der ersten Fläche (130) gegenüberliegt, und
wobei die Positioniermittel durch ein Loch (221) ausgebildet werden, das durch die erste Fläche (130) in Richtung der zweiten Fläche (130) führt.

18. Körperfettmessvorrichtung gemäß Anspruch 12, wobei
der Hauptabschnitt (110) weiterhin eine zweite Fläche (130B) aufweist, die der ersten Fläche (130) gegenüberliegt, und
wobei die Positioniermittel durch ein Fenster (221) ausgebildet sind, das eine Sicht von der zweiten Fläche (130B) aus in Richtung der ersten Fläche (130) zur Verfügung stellt.

**Revendications**

1.  Appareil de mesure de graisse corporelle comprenant :

    une unité de membre supérieur (1) comprenant une partie de maintien (121, 122) qui est adaptée pour être maintenue dans une main d'un sujet, une partie d'affichage (21) pour afficher une quantité de graisse viscérale, une partie d'entrée (22) pour entrer des données physiques dudit sujet et une partie principale (110), ladite partie de maintien (121, 122) comprenant une électrode de membre supérieur (H1, H2, H3, H4) qui est adaptée pour être en contact avec une partie de paume dudit sujet, ladite partie principale (110) comprenant une première surface (130) qui est adaptée pour être en contact avec une surface d'un abdomen dudit sujet, et un groupe d'électrodes abdominales (A, B, C, D) qui sont agencées au niveau de ladite première surface (130), et ledit groupe d'électrodes abdominales (A, B, C, D) comprenant une paire de premières électrodes (B, C) et une paire de secondes électrodes (A, D), ledit appareil de mesure de graisse corporelle comprenant en outre :

        une électrode de membre inférieur (F1, F2, F3, F4) qui est adaptée pour être en contact avec un membre inférieur dudit sujet ;
        une partie d'application (12, 14) pour appliquer un courant électrique sur ledit sujet via une paire d'électrodes consistant en ladite électrode de membre supérieur (H1, H2, H3, H4) et de ladite électrode de membre inférieur (F1, F2, F3, F4) et via la paire desdites secondes électrodes (A, D) ;
        une partie de détection (13) pour détecter deux types de différences de potentiel respectivement dans un premier cas dans lequel le courant électrique est appliqué via la paire composée de ladite électrode de membre supérieur (H1, H2, H3, H4) et de ladite électrode de membre inférieur (F1, F2, F3, F4) et dans un second cas dans lequel le courant électrique est appliqué via la paire desdites secondes électrodes (A, D), ladite partie de détection (13) détectant une première différence de potentiel entre les électrodes d'une paire prédéterminée desdites première ou seconde électrodes dans ledit premier cas, et une seconde différence de potentiel entre lesdites premières électrodes (A, D) dans ledit second cas ; et
        une partie de calcul de quantité de graisse viscérale (262A) pour calculer une quantité de graisse viscérale dudit sujet en fonction de ladite première différence de potentiel et de ladite seconde différence de potentiel qui sont détectées et des données physiques dudit sujet,
        ladite partie d'affichage (21) étant adaptée pour afficher ladite quantité de graisse viscérale calculée.

2.  Appareil de mesure de graisse corporelle selon la revendication 1, dans lequel
    lesdites premières électrodes (B, C) et lesdites secondes électrodes (A, D) sont agencées au niveau de ladite première surface (130) dans une direction d'alignement qui est une direction verticale dans un état dans lequel ledit sujet prend une posture pour prendre une mesure et tient ladite partie de maintien (121, 122).

3.  Appareil de mesure de graisse corporelle selon la revendication 2, dans lequel
    lesdites secondes électrodes (A, D) sont agencées dans des positions respectives de sorte que lesdites premières électrodes (B, C) sont agencées entre lesdites secondes électrodes (A, D) dans ladite direction d'alignement.

4.  Appareil de mesure de graisse corporelle selon la revendication 2, dans lequel
    les électrodes de ladite paire prédéterminée sont lesdites premières électrodes (B, C).

5.  Appareil de mesure de graisse corporelle selon la revendication 2, dans lequel
    les électrodes de ladite paire prédéterminée sont une paire d'électrodes différentes de la paire desdites premières électrodes (B, C) incluses dans ledit groupe d'électrodes abdominales.

6.  Appareil de mesure de graisse corporelle selon la revendication 5, dans lequel
    lesdites secondes électrodes (A, D) sont agencées dans des positions respectives de sorte que lesdites premières électrodes (B, C) sont agencées entre lesdites secondes électrodes (A, D) dans ladite direction d'alignement, et
    les électrodes de ladite paire prédéterminée sont lesdites secondes électrodes (A, D).

7.  Appareil de mesure de graisse corporelle selon la revendication 1, comprenant en outre une partie de calcul d'impédance (261) pour calculer deux types d'impédances en fonction de ladite première différence de potentiel et de ladite seconde différence de potentiel respectivement, dans lequel

ladite partie de calcul de quantité de graisse viscérale (262A) calcule ladite quantité de graisse viscérale en fonction desdits deux types d'impédances qui sont calculés et desdites données physiques dudit sujet.

8. Appareil de mesure de graisse corporelle selon la revendication 7, dans lequel
ladite partie de calcul de graisse viscérale (262A) calcule ladite quantité de graisse viscérale en utilisant une formule de corrélation prédéterminée d'une relation entre lesdits deux types d'impédances, lesdites données physiques et la quantité de graisse viscérale.

9. Appareil de mesure de graisse corporelle selon la revendication 1, comprenant en outre une partie de calcul de quantité de graisse sous-cutanée (262B) pour calculer une quantité de graisse sous-cutanée dudit sujet en fonction de ladite seconde différence de potentiel telle que détectée et desdites données physiques dudit sujet.

10. Appareil de mesure de graisse corporelle selon la revendication 1, dans lequel
lesdites données physiques comprennent au moins l'un parmi la circonférence de l'abdomen, la largeur latérale de l'abdomen, l'épaisseur de l'abdomen, la taille et le poids dudit sujet.

11. Appareil de mesure de graisse corporelle selon la revendication 1, comprenant en outre une unité de membre inférieur (2) qui est adaptée de sorte qu'un pied dudit sujet peut être placé sur cette dernière, dans lequel
ladite électrode de membre inférieur (F1, F2, F3, F4) est prévue au niveau de ladite unité de membre inférieur (2).

12. Appareil de mesure de graisse corporelle selon la revendication 1, dans lequel
ladite partie principale (110) comprend en outre des moyens de positionnement (201, 203, 206, 211, 212, 221) qui sont adaptés pour être agencés dans une position correspondant à une position ombilicale dudit sujet.

13. Appareil de mesure de graisse corporelle selon la revendication 12, dans lequel
lesdits moyens de positionnement sont formés par une dépression (201) prévue dans une partie d'extrémité supérieure de ladite première surface (130).

14. Appareil de mesure de graisse corporelle selon la revendication 12, dans lequel
lesdits moyens de positionnement comprennent
une caméra (203) prévue au niveau de ladite première surface (130), et
dans lequel la partie d'affichage (21) comprend une région prédéterminée (21A) pour afficher un objet dont l'image est prise par ladite caméra (203).

15. Appareil de mesure de graisse corporelle selon la revendication 12, dans lequel
lesdits moyens de positionnement sont formés par un miroir (212) prévu au niveau de ladite première surface (130).

16. Appareil de mesure de graisse corporelle selon la revendication 12, dans lequel
lesdits moyens de positionnement sont formés par un appareil de rayonnement (206) prévu au niveau de ladite première surface (130) pour appliquer de la lumière laser.

17. Appareil de mesure de graisse corporelle selon la revendication 12, dans lequel
ladite partie principale (110) comprend en outre une seconde surface (130B) opposée à ladite première surface (130), et
lesdits moyens de positionnement sont formés par un trou (221) passant par ladite première surface (130) vers ladite seconde surface (130).

18. Appareil de mesure de graisse corporelle selon la revendication 12, dans lequel
ladite partie principale (110) comprend en outre une seconde surface (130B) opposée à ladite première surface (130), et
lesdits moyens de positionnement sont formés par une fenêtre (221) fournissant la visibil-té de ladite seconde surface (130B) vers ladite première surface (130).

FIG.1

FIG.2A

FIG.2B

FIG.3

# FIG.4

26

OPERATION PROCESSING PORTION

261

IMPEDANCE CALCULATING PORTION

262

BODY FAT CALCULATING PORTION

VISCERAL FAT AMOUNT CALCULATING PORTION — 262A

SUBCUTANEOUS FAT AMOUNT CALCULATING PORTION — 262B

BODY FAT AMOUNT CALCULATING PORTION — 262C

# FIG.5

## FIG.6

START

RECEIVE INPUT OF SUBJECT DATA — S2

INSTRUCTION TO MEASURE GIVEN? — S4
NO
YES

SET ELECTRODES — S6

APPLY CURRENT FROM ELECTRODES H1 AND H2 TO ELECTRODES F1 AND F2 — S8

DETECT POTENTIAL DIFFERENCES BETWEEN ELECTRODES H3, H4 AND ELECTRODES F3, F4 — S10

CALCULATE IMPEDANCE Zw — S12

SWITCH ELECTRODES FOR VOLTAGE — S14

DETECT POTENTIAL DIFFERENCE BETWEEN ELECTRODES B-C — S16

CALCULATE IMPEDANCE Zt — S17

SWITCH ELECTRODES FOR CURRENT — S19

APPLY CURRENT BETWEEN ELECTRODES A-D — S20

DETECT POTENTIAL DIFFERENCE BETWEEN ELECTRODES B-C — S22

CALCULATE IMPEDANCE Zs — S24

CALCULATE VISCERAL FAT AREA — S26

CALCULATE SUBCUTANEOUS FAT AREA — S28

CALCULATE BODY FAT PERCENTAGE — S30

DISPLAY MEASUREMENT RESULTS — S32

END

## FIG.7

VISCERAL FAT AREA          $110cm^2$

SUBCUTANEOUS FAT AREA    $90cm^2$

BODY FAT PERCENTAGE       30%

21

## FIG.8A

VISCERAL FAT AREA          $110cm^2$

21

## FIG.8B

VISCERAL FAT AREA          $110cm^2$

SUBCUTANEOUS FAT AREA    $90cm^2$

21

## FIG.9A

21

PATIENT ID   012345      WAIST LENGTH    80cm
       AGE   35              HEIGHT    170cm
  GENDER   MALE            WEIGHT     80kg

VISCERAL FAT AREA          $110cm^2$

SUBCUTANEOUS FAT AREA   $90cm^2$

BODY FAT PERCENTAGE      30%

## FIG.9B

21

PATIENT ID   012345      WAIST LENGTH    80cm
       AGE   35              HEIGHT    170cm
  GENDER   MALE            WEIGHT     80kg

VISCERAL FAT AREA          $110cm^2$

BODY FAT PERCENTAGE      30%

## FIG.10A

21

| VISCERAL FAT AREA | 110cm$^2$ | REFERENCE VALUE | 100cm$^2$ |
|---|---|---|---|
| SUBCUTANEOUS FAT AREA | 90cm$^2$ | | |
| BODY FAT PERCENTAGE | 30% | STANDARD VALUE | 10~20% |

## FIG.10B

21

YOUR

STANDARD VALUE OF THE MALE OF YOUR AGE

| VISCERAL FAT AREA | 110cm$^2$ | VISCERAL FAT AREA | 110cm$^2$ |
|---|---|---|---|
| SUBCUTANEOUS FAT AREA | 90cm$^2$ | SUBCUTANEOUS FAT AREA | 90cm$^2$ |
| BODY FAT PERCENTAGE | 30% | BODY FAT PERCENTAGE | 30% |

## FIG.11

21

AREA
[cm$^2$]

L1

L3

100

L2

PRESENT VALUE

| VISCERAL FAT AREA | 110cm$^2$ | SUBCUTANEOUS FAT AREA | 90cm$^2$ |
|---|---|---|---|

FIG.12A

FIG.12B

FIG.13

FIG.14

EP 1 958 567 B1

## FIG.15

```
                    ( START )
                        |
                        v
        RECEIVE INPUT OF SUBJECT DATA          — S2
                        |
                        v
  NO   <  INSTRUCTION TO MEASURE GIVEN?  >      — S4
                        |
                       YES
                        v
              SET ELECTRODES                    — S6
                        |
                        v
   APPLY CURRENT FROM ELECTRODES H1             — S8
   AND H2 TO ELECTRODES F1 AND F2
                        |
                        v
   DETECT POTENTIAL DIFFERENCES BETWEEN         — S10
   ELECTRODES H3, H4 AND ELECTRODES F3, F4
                        |
                        v
         CALCULATE IMPEDANCE Zw                 — S12
                        |
                        v
      SWITCH ELECTRODES FOR VOLTAGE             — S14A
                        |
                        v
   DETECT POTENTIAL DIFFERENCE BETWEEN          — S16A
   ELECTRODES B11–C11
                        |
                        v
         CALCULATE IMPEDANCE Zt1                — S17A
                        |
                        v
      SWITCH ELECTRODES FOR VOLTAGE             — S181
                        |
                        v
   DETECT POTENTIAL DIFFERENCE BETWEEN          — S182
   ELECTRODES B12–C12
                        |
                        v
         CALCULATE IMPEDANCE Zt2                — S183
                        |
                        v
                      ( A )
```

# FIG.16

```
                    ( A )
                      │
                      ▼
┌─────────────────────────────────────┐
│ SWITCH ELECTRODES FOR CURRENT AND    │── S19A
│ ELECTRODES FOR VOLTAGE               │
└─────────────────────────────────────┘
                      │
                      ▼
┌─────────────────────────────────────┐
│ APPLY CURRENT BETWEEN ELECTRODES     │── S20A
│ A11-D11                              │
└─────────────────────────────────────┘
                      │
                      ▼
┌─────────────────────────────────────┐
│ DETECT POTENTIAL DIFFERENCE          │── S22A
│ BETWEEN ELECTRODES B11-C11           │
└─────────────────────────────────────┘
                      │
                      ▼
┌─────────────────────────────────────┐
│ CALCULATE IMPEDANCE Zs1              │── S24A
└─────────────────────────────────────┘
                      │
                      ▼
┌─────────────────────────────────────┐
│ SWITCH ELECTRODES FOR CURRENT AND    │── S251
│ ELECTRODES FOR VOLTAGE               │
└─────────────────────────────────────┘
                      │
                      ▼
┌─────────────────────────────────────┐
│ APPLY CURRENT BETWEEN ELECTRODES     │── S252
│ A12-D12                              │
└─────────────────────────────────────┘
                      │
                      ▼
┌─────────────────────────────────────┐
│ DETECT POTENTIAL DIFFERENCE          │── S253
│ BETWEEN ELECTRODES B12-C12           │
└─────────────────────────────────────┘
                      │
                      ▼
┌─────────────────────────────────────┐
│ CALCULATE IMPEDANCE Zs2              │── S254
└─────────────────────────────────────┘
                      │
                      ▼
┌─────────────────────────────────────┐
│ CALCULATE VISCERAL FAT AREA          │── S26A
└─────────────────────────────────────┘
                      │
                      ▼
┌─────────────────────────────────────┐
│ CALCULATE SUBCUTANEOUS FAT AREA      │── S28A
└─────────────────────────────────────┘
                      │
                      ▼
┌─────────────────────────────────────┐
│ CALCULATE BODY FAT PERCENTAGE        │── S30
└─────────────────────────────────────┘
                      │
                      ▼
┌─────────────────────────────────────┐
│ DISPLAY MEASUREMENT RESULTS          │── S32
└─────────────────────────────────────┘
                      │
                      ▼
              (   END   )
```

FIG.17A

1A

111

112A

E1    211    E2    130A

FIG.17B

1A

111

112A

212

211    130A

FIG.18

111    110B
112B

1B

H1    H3    H4    H2

121    122

A21    C21
B21    D21
A22    C22
B22    D22

130B

FIG.19

EP 1 958 567 B1

## FIG.20

```
                    ( START )
                        │
                        ▼
        ┌─────────────────────────────────┐
        │   RECEIVE INPUT OF SUBJECT DATA  │──S2
        └─────────────────────────────────┘
                        │
              ┌─────────▼──────── S4
        NO   ╱                            ╲
        ◄───◄   INSTRUCTION TO MEASURE GIVEN?  ►
              ╲                            ╱
                        │
                     ▼ YES
        ┌─────────────────────────────────┐
        │          SET ELECTRODES         │──S6
        └─────────────────────────────────┘
                        │
                        ▼
        ┌─────────────────────────────────┐
        │ APPLY CURRENT FROM ELECTRODES H1 AND H2 TO │──S8
        │ ELECTRODES F1 AND F2            │
        └─────────────────────────────────┘
                        │
                        ▼
        ┌─────────────────────────────────┐
        │ DETECT POTENTIAL DIFFERENCES BETWEEN │──S10
        │ ELECTRODES H3, H4 AND ELECTRODES F3, F4 │
        └─────────────────────────────────┘
                        │
                        ▼
        ┌─────────────────────────────────┐
        │      CALCULATE IMPEDANCE Zw      │──S12
        └─────────────────────────────────┘
                        │
                        ▼
        ┌─────────────────────────────────┐
        │   SWITCH ELECTRODES FOR VOLTAGE  │──S14B
        └─────────────────────────────────┘
                        │
                        ▼
        ┌─────────────────────────────────┐
        │ DETECT POTENTIAL DIFFERENCE BETWEEN │──S16B
        │ ELECTRODES A21–A22              │
        └─────────────────────────────────┘
                        │
                        ▼
        ┌─────────────────────────────────┐
        │      CALCULATE IMPEDANCE Zt      │──S17
        └─────────────────────────────────┘
                        │
                        ▼
        ┌─────────────────────────────────┐
        │ SWITCH ELECTRODES FOR CURRENT AND ELECTRODES │──S19B
        │ FOR VOLTAGE                     │
        └─────────────────────────────────┘
                        │
                        ▼
        ┌─────────────────────────────────┐
        │ APPLY CURRENT FROM ELECTRODES A21 AND A22 TO │──S20B
        │ ELECTRODES D21 AND D22          │
        └─────────────────────────────────┘
                        │
                        ▼
        ┌─────────────────────────────────┐
        │ DETECT POTENTIAL DIFFERENCES BETWEEN │──S22B
        │ ELECTRODES B21, B22 AND ELECTRODES C21, C22 │
        └─────────────────────────────────┘
                        │
                        ▼
        ┌─────────────────────────────────┐
        │      CALCULATE IMPEDANCE Zs      │──S24
        └─────────────────────────────────┘
                        │
                        ▼
        ┌─────────────────────────────────┐
        │    CALCULATE VISCERAL FAT AREA   │──S26
        └─────────────────────────────────┘
                        │
                        ▼
        ┌─────────────────────────────────┐
        │ CALCULATE SUBCUTANEOUS FAT AREA  │──S28
        └─────────────────────────────────┘
                        │
                        ▼
        ┌─────────────────────────────────┐
        │  CALCULATE BODY FAT PERCENTAGE   │──S30
        └─────────────────────────────────┘
                        │
                        ▼
        ┌─────────────────────────────────┐
        │   DISPLAY MEASUREMENT RESULTS    │──S32
        └─────────────────────────────────┘
                        │
                        ▼
                    (  END  )
```

FIG.21

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7079938 A
- JP 2000152919 A
- JP 2002369806 A
- JP 2002282241 A
- EP 1707117 A
- EP 1731092 A
- EP 1741385 A